(19) Europäisches Patentamt
European Patent Office
Office européen des brevets

(11) **EP 4 285 931 A1**

(12) **EUROPEAN PATENT APPLICATION**
published in accordance with Art. 153(4) EPC

(43) Date of publication:
06.12.2023 Bulletin 2023/49

(21) Application number: 22745913.8

(22) Date of filing: 26.01.2022

(51) International Patent Classification (IPC):
*A61K 47/32* (2006.01)   *A61K 8/34* (2006.01)
*A61K 8/37* (2006.01)   *A61K 8/41* (2006.01)
*A61K 8/73* (2006.01)   *A61K 8/81* (2006.01)
*A61K 9/06* (2006.01)   *A61K 9/08* (2006.01)
*A61K 9/10* (2006.01)   *A61K 9/107* (2006.01)
*A61K 9/12* (2006.01)   *A61K 31/045* (2006.01)
*A61K 31/165* (2006.01)   *A61K 31/192* (2006.01)
*A61K 31/235* (2006.01)   *A61K 31/618* (2006.01)
*A61K 36/534* (2006.01)   *A61K 36/61* (2006.01)
*A61K 36/756* (2006.01)   *A61K 36/81* (2006.01)
*A61K 45/00* (2006.01)

(52) Cooperative Patent Classification (CPC):
**A61K 8/34; A61K 8/37; A61K 8/41; A61K 8/73;
A61K 8/81; A61K 9/06; A61K 9/08; A61K 9/10;
A61K 9/107; A61K 9/12; A61K 31/045;
A61K 31/165; A61K 31/192; A61K 31/235;
A61K 31/618;**   (Cont.)

(86) International application number:
**PCT/JP2022/002876**

(87) International publication number:
**WO 2022/163694 (04.08.2022 Gazette 2022/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 29.01.2021   JP 2021013554
17.09.2021   JP 2021152516

(71) Applicant: Kao Corporation
**Chuo-ku
Tokyo 103-8210 (JP)**

(72) Inventors:
• **UCHIDA, Takashi**
**Tokyo 131-8501 (JP)**
• **NOHARA, Makoto**
**Tokyo 131-8501 (JP)**
• **OKUBO, Emi**
**Tokyo 131-8501 (JP)**
• **OSUMI, Shinzo**
**Tokyo 131-8501 (JP)**
• **FUJII, Ryosuke**
**Wakayama-shi, Wakayama 640-8580 (JP)**

(74) Representative: **Hoffmann Eitle
Patent- und Rechtsanwälte PartmbB
Arabellastraße 30
81925 München (DE)**

(54) **COMPOSITION FOR EXTERNAL APPLICATION**

(57)    Provided is a composition for external application, containing (A) a medicinal ingredient, (B) a water-insoluble polymer, (C) a non-volatile base, (D) a volatile solvent, and (E) water, in which the composition has a viscosity at 25°C of 1.0 mPa ·s or more and 10,000 mPa ·s or less.

(52) Cooperative Patent Classification (CPC): (Cont.)
**A61K 36/534; A61K 36/61; A61K 36/756;**
**A61K 36/81; A61K 45/00; A61K 47/10;**
**A61K 47/14; A61K 47/18; A61K 47/28;**
**A61K 47/32; A61K 47/38; A61P 9/08;**
**A61P 17/00; A61P 17/04; A61P 23/02;**
**A61P 25/02; A61P 29/00; A61P 31/04;**
**A61P 43/00; A61Q 19/00**

**Description**

Field of the Invention

The present invention relates to a composition for external application.

Background of the Invention

A liquid skin external application and a patch containing a medicinal component such as an anti-inflammatory analgesic component and a bactericidal disinfectant component are known. However, existing liquid skin external applications have problems that they are easily rubbed off by clothes or the like after being applied to the skin, and it is difficult to sustainably release the medicinal ingredients into the skin. In the case of a patch containing a medicinal ingredient, when the patch does not easily follow the skin and is easily peeled off, it is difficult to sustainably release the medicinal ingredient to the skin. On the other hand, a patch having high adhesiveness to the skin also has a problem that a burden on the skin increases when the patch is peeled off from the skin. In addition, a patch has problems in practical use such as a tendency to cause rash and a concern about the appearance when the patch is attached to the skin.

Therefore, a film-forming formulation capable of sustainably releasing a medicinal ingredient into the skin has been investigated. Since a film-forming formulation can form a coating film when applied to the skin, it is less likely to rub off than a liquid skin external application. In addition, even in a case where the film-forming formulation is applied to the skin, it does not stand out as in a patch, and it is preferable from the viewpoint that the burden on the skin is small in a case where the film-forming formulation is removed from the skin.

For example, PTL 1 (JP 2009-519956 A) proposes an adhesive solid formulation for skin delivery of a drug, the formulation containing: a drug; a solvent excipient including a volatile solvent system containing a volatile solvent and a non-volatile solvent system containing a non-volatile solvent; and a solidifying agent, in which the formulation has a viscosity suitable for application and adhesion to a skin surface prior to evaporation of the volatile solvent system, in which the formulation applied to the skin surface forms a solidified layer after at least partial evaporation of the volatile solvent system, in which the drug is continuously delivered to the skin after at least substantial evaporation of the volatile solvent system, provided that at least two volatile solvents, at least two non-volatile solvents, or at least two solidifying agents are present. It is described that the formulation can provide sustained drug delivery over an extended period of time, is not vulnerable to unintended removal by contact with clothing, other objects, or people for the duration of the application time, can be applied to skin areas subject to stretching and expansion without causing uncomfortable or poor contact with the skin, and can be easily removed after application and use.

PTL 2 (JP 2014-515365 A) proposes a composition for transdermal administration of a physiologically active agent containing: an ethyl acrylate/methyl methacrylate copolymer; ethanol, isopropanol or a mixture thereof; water; and at least one physiologically active agent. It is described that the composition is a film-forming composition that rapidly forms a non-sticky, durable, and very soft film.

The present invention relates to the following.

[1] A composition for external application, containing (A) a medicinal ingredient, (B) a water-insoluble polymer, (C) a non-volatile base, (D) a volatile solvent, and (E) water, in which the composition has a viscosity at 25°C of 1.0 mPa s or more and 10,000 mPa ·s or less.

[2] A lotion formulation, a gel formulation, an ointment formulation, a cream formulation, or a foam formulation containing the composition for external application as set forth in [1].

[3] An aerosol formulation or a pump spray-type formulation using the composition for external application as set forth in [1].

[4] A method for using a composition for external application, including a step of applying to the skin a composition for external application containing (A) a medicinal ingredient, (B) a water-insoluble polymer, (C) a non-volatile base, (D) a volatile solvent, and (E) water, in which the composition has a viscosity at 25°C of 1.0 mPa.s or more and 10,000 mPa s or less, and then drying the composition.

[5] A method for producing a coating film, including a step of applying the composition for external application as set forth in [1] to the skin by direct application or spraying.

Detailed Description of the Invention

[Composition for External Application]

The present invention relates to a composition for external application, containing (A) a medicinal ingredient, (B) a water-insoluble polymer, (C) a non-volatile base, (D) a volatile solvent, and (E) water, in which the composition has a viscosity at 25°C of 1.0 mPa ·s or more and 10,000 mPa s or less. Hereinafter, the composition is also appropriately referred to as "the composition of the present invention".

<Definitions>

In the description herein, the term "composition for external application" refers to a composition mainly applied to skin surfaces.

In the description herein, the term "water-insoluble polymer" refers to a polymer in which 1 g of the polymer is immersed in 10 g of ion-exchange water under an environment of 23°C and 1 atmosphere, and after 24 hours have elapsed, more than 0.5 g of the immersed polymer does not dissolve.

In the description herein, the term "non-volatile base" refers to a base that is in a liquid state at 70°C, which has a mass loss rate of less than 1% when 1 g of the base is spread on a glass Petri dish having a diameter of 48 mm and allowed to stand at 25°C and atmospheric pressure for 24 hours.

The term "volatile solvent" refers to a component other than water that is liquid at 25°C and has a mass loss rate of 1% or more when 1 g of the solvent is spread on a glass Petri dish having a dimeter of 48 mm and allowed to stand at 25°C and atmospheric pressure for 24 hours.

In addition, in the description herein, the resistance of the coating film to sweat, humidity, and the like (moisture resistance) and the resistance of the coating film to being rubbed off (rub fastness) are collectively referred to as "durability of the coating film".

The composition of the present invention contains a component (A): a medicinal ingredient, a component (B): a water-insoluble polymer, a component (C): a non-volatile base, a component (D): a volatile base, and a component (E): water, but the component (A) is a component not contained in any of the other components (B), (C), and (D), and the component (B) is a component not contained in either the component (C) or the component (D).

In the techniques disclosed in PTLs 1 and 2, there is room for improvement in sustainedly release of a drug, a physiologically active agent, or the like to the skin in a sustained manner. In addition, it is also desired to improve performances in practical use as a composition for external application, such as quick-drying property after application of the composition to the skin and appearance.

The present invention relates to a composition for external application which can sustainedly release a medicinal ingredient to the skin in a sustained manner, has good film-forming property, high coating property to the skin, and high quick-drying property, and is excellent in the effect of suppressing stickiness and tense feeling of a coating film to be formed, and the appearance and durability of the coating film.

The present inventors have found that the above-mentioned problems can be solved by a composition for external application containing a medicinal ingredient, a water-insoluble polymer, and a predetermined solvent component and having a predetermined viscosity.

According to the present invention, it is possible to provide a composition for external application which can sustainedly release a medicinal ingredient to the skin in a sustained manner, has good film-forming property, high coating property to the skin, and high quick-drying property, and is excellent in the effect of suppressing stickiness and tense feeling of a coating film to be formed, and the appearance and durability of the coating film, a formulation using the same, a method of using a composition for external application, and a method of producing a coating film.

Since the composition of the present invention has the above-mentioned constitution, the composition can sustainedly release the medicinal ingredient to the skin in a sustained manner, has good film-forming property, high coating property to the skin, and high quick-drying property, and is excellent in the effect of suppressing the stickiness and tense feeling of the coating film to be formed, and the appearance and durability of the coating film. The reason is not certain, but is considered as follows.

The composition of the present invention is a composition having film-forming property, which contains, as film-forming components, a water-insoluble polymer as the component (B) and a non-volatile base as the component (C). When the composition of the present invention is applied to the skin and then dried, the component (D) and the component (E) volatilize to form a coating film containing the components (A) to (C).

Here, when a highly crystalline ingredient such as felbinac, which will be described later, is used as the medicinal ingredient which is the component (A), if the compatibility between the component (A) and the coating film is low, the component (A) is likely to be crystallized in the coating film, and the sustained release to the skin is reduced. However, even if the compatibility between the component (A) and the coating film is too high, it is considered that the component (A) is taken into the coating film and is difficult to be sustained-released to the skin side.

It is considered that, in a case where the water-insoluble polymer which is the component (B) is used as the film-forming component, the compatibility between the coating film and the component (A) becomes excessively high or low, and the sustained release of the component (A) may be reduced. In the composition of the present invention, it is considered that the use of the component (C) reduces the compatibility between the coating film and the component (A) to suppress the crystallization of the component (A) when the compatibility between the coating film and the component (A) is excessively high, or improves the compatibility between the coating film and the component (A) to suppress the crystallization of the component (A) when the compatibility between the coating film and the component (A) is low, thereby improving the sustained release of the component (A).

Further, since the component (B) is water-insoluble, a highly hydrophobic coating film can be formed, and thus, even after application to the skin, the coating film is hardly rubbed off due to sweat, humidity, or the like, and it is possible to sustainedly release the medicinal ingredient into the skin in a sustained manner. Furthermore, due to the synergistic effect of the components (B) and (C), it is considered that it is possible to obtain a composition capable of forming a coating film which has high coating property to the skin and quick-drying property, does not have stickiness and a tense feeling, has a good appearance, is less likely to be rubbed off even after application to the skin, and has good durability.

It is considered that the component (D) contributes to the improvement of the coating property, quick-drying property, storage stability, and the like of the composition, and the component (E) contributes to the improvement of the storage stability of the composition. In addition, it is considered that by containing the components (D) and (E) and setting the viscosity within a predetermined range, the composition of the present invention becomes easily compatible with the skin, the film-forming property and the durability of the coating film can be compatible with the coating property and the quick-drying property, and as a result, the component (A) can be effectively and sustained-released into the skin.

<Component (A): Medicinal Ingredient>

The composition of the present invention contains a medicinal ingredient as the component (A). From the viewpoint of use in the composition for external application, it is preferable that the component (A) is a skin external drug ingredient that can be administered transdermally. Specifically, it is preferable that the component (A) contains one or more selected from the group consisting of an anti-inflammatory analgesic component, a local irritation component, a blood circulation promoting component, an antihistamine component, a crude drug component, an antipruritic component, a local anesthetic component, a keratin softening component, a bactericidal component, and an antibacterial component.

Examples of the anti-inflammatory analgesic component include glycol salicylate, methyl salicylate, aspirin, sulpyrine hydrate, acetaminophen, diclofenac sodium, fenbufen, ibuprofen, alminoprofen, loxoprofen sodium or a hydrate thereof, naproxen, oxaprofen, ketoprofen, tiaprofenic acid, sulindac, flufenamate aluminum, felbinac, mefenamic acid, indometacin, indometacin farnesil, acemetacin, proglumetacin maleate, bendazac, piroxicam, ampiroxicam, lornoxicam, tenoxicam, meloxicam, etodolac, tiaramide hydrochloride, bucolome, flurbiprofen, esflurbiprofen, lysozyme hydrochloride, bromelain, diphenhydramine hydrochloride, dibucaine, 1,4-dimethyl-7-isopropylazulene, benzethonium chloride, glycyrrhizic acid, dipotassium glycyrrhizinate, zincoxide, allantoin, heparinoid, and glycyrrhetinic acid.

Examples of the local irritation component include l-menthol, dl-camphor, nonylic acid vanillylamide, ammonia, and peppermint oil.

Examples of the blood circulation promoting component include benzyl nicotinate, sodium polyethylenesulfonate, and tocopherol acetate.

Examples of the antihistaminic component include diphenhydramine, diphenhydramine hydrochloride, diphenhydramine salicylate, and chlorpheniramine maleate.

Examples of the crude drug component include capsicum, eucalyptus oil, phellodendron bark, arnica tincture, houttuynia herb, peony root, magnolol, cinnamon bark, cnidium rhizome, cyperus rhizome, atractylodes lancea rhizome, illicium verum, schisandra repanda, citrus unshiu peel, ginseng, mangrove bark, clove, ginger, gardenia fruit, glycyrrhiza, fennel, akebia stem, safflower, magnolia bark, bitter orange peel, and artemisia capillaris flower.

Examples of the antipruritic ingredient include crotamiton, cortisone acetate, isothipendyl hydrochloride, benzalkonium chloride, calamine, d-borneol, ammonia water, hydrocortisone, hydrocortisone acetate, hydrocortisone butyrate, dexamethasone, dexamethasone acetate, prednisolone, prednisolone acetate, prednisolone valerate acetate, and ufenamate.

Examples of the local anesthetic component include lidocain, mepivacaine, bupivicaine, ropivacaine, levobupivacaine, dibucaine, dibucaine hydrochloride, and ethyl aminobenzoate.

Examples of the keratin softening component include urea, sulfur, and salicylic acid.

Examples of the bactericidal component include chlorohexidine gluconate, sodium copper chlorophyllin, isopropylmethylphenol, cetylpyridinium chloride hydrate, benzethonium chloride, benzalkonium chloride, resorcinol, acrinol hydrate, chlorohexidine gluconate, povidone iodine, iodine-potassium iodide, mercurochrome, oxydol, cresol, iodoform, and thymol.

Examples of the antibacterial component include undecylenic acid, zinc undecylenate, phenyl-11-iodo-10-undecenoate, exalamide, clotrimazole, econazole nitrate, miconazole nitrate, tioconazole, zinc diethyldithiocarbamate, ciclopiroxolamine, siccanin, trichomycin, pyrrolnitrin, thianthol, 2,4,6-tribromophenyl caproate, trimethylcetylammonium pentachlorophenate, tolciclate, tolnaftate, haloprogin, althea bark, berberine benzoate, dequalinium chloride, chlorhexidine hydrochloride, chlorhexidine gluconate solution, dequalinium acetate, hinokitiol, resorcin, benzoic acid, chlorobutanol, acetic acid, phenol, iodine tincture, diphenylpyraline hydrochloride, diphenhydramine salicylate, diphenylimidazole, chlorpheniramine maleate, dibucaine hydrochloride, procaine hydrochloride, lidocaine hydrochloride, aldioxa, glycyrrhizic acid and its salts, lithospermum root, Angelica acutiloba, borneol, diethyl phthalate, chlorohydroxy aluminum, penicillin, cephem, carbapenem, monobactam, penam, aminoglycoside, fosfomycin, chloramphenicol, macrolide, glycopeptide, quinolone, new quinolone, sulfa drug, fradiomycin sulfate, gentamicin sulfate, pentamidine isethionate, silver sulfadiazine, oxiconazole nitrate, sulconazole nitrate, bifonazole, neticonazole hydrochloride, lanoconazole, butenafine hydrochloride, amorolfine hydrochloride, and terbinafine hydrochloride.

As the component (A), one or more of the above-mentioned medicinal ingredients can be used.

Among the above, from the viewpoint that the component (A) is a composition for external application used for an exposed portion and a bent portion of the skin, the component (A) preferably contains one or more selected from the group consisting of an anti-inflammatory analgesic component, a local irritation component, a blood circulation promoting component, an antihistamine component, and a crude drug component, and more preferably contains one or more selected from the group consisting of an anti-inflammatory analgesic component and a local irritation component. Specifically, the component (A) more preferably contains one or more selected from the group consisting of glycol salicylate, methyl salicylate, diclofenac sodium, loxoprofen sodium, ketoprofen, felbinac, indomethacin, piroxicam, flurbiprofen, glycyrrhizic acid, glycyrrhetinic acid, l-menthol, dl-camphor, nonylic acid vanillylamide, peppermint oil, benzyl nicotinate, sodium polyethylene sulfonate, tocopherol acetate, diphenhydramine, chlorpheniramine maleate, capsicum, eucalyptus oil, and phellodendron bark.

The solubility parameter (SP value) of the component (A) is preferably 15.00 or more, more preferably 18.00 or more, still more preferably 19.00 or more, and even more preferably 19.50 or more, from the viewpoint that the coating film to be formed has a good appearance, and from the viewpoint that a tense feeling of the coating film to be formed is suppressed. On the other hand, from the same viewpoint, it is preferably 35.00 or less, more preferably 27.00 or less, still more preferably 24.00 or less, and even more preferably 22.00 or less. Thus, the SP value of the component (A) is preferably 15.00 or more and 35.00 or less, more preferably 18.00 or more and 27.00 or less, still more preferably 19.00 or more and 24.00 or less, and even more preferably 19.50 or more and 22.00 or less.

The SP value can be calculated by a solubility parameter calculation software (Hansen Solubility Parameters in Practice 4th Edition 4.1.03). In the description herein, the SP value is calculated based on the Hansen solubility parameter. The Hansen solubility parameter is obtained by dividing the Hildebrand solubility parameter into three components of a dispersion term $\delta D$, a polarity term $\delta P$, and a hydrogen bonding term $\delta H$, and representing the three components in a three dimensional space. Specifically, the SP value $\delta$ is calculated using the following equation.

$$\delta = (\delta D^2 + \delta P^2 + \delta H^2)^{1/2}$$

The SP value in a mixture of two or more components can be calculated in the following manner.

For example, assuming that a mixture of two components of Component 1 and Component 2 is "Mixture X", the solubility parameters of Hansen of Component 1 and Component 2 constituting Mixture X are [$\delta D1$, $\delta P1$, $\delta H1$] and [$\delta D2$, $\delta P2$, $\delta H2$], respectively, and the volume ratio of Component 1 to Component 2 is V1:V2, the dispersion term $\delta Dm$, the polarity term $\delta Pm$, and the hydrogen bonding term $\delta Hm$ of Mixture X are represented by the following equations. In the case of a polymer, the specific gravity was calculated as 1.0.

$$\delta Dm = (V1 \times \delta D1 + V2 \times \delta D2)/(V1 + V2)$$

$$\delta Pm = (V1 \times \delta P1 + V2 \times \delta P2)/(V1 + V2)$$

$$\delta Hm = (V1 \times \delta H1 + V2 \times \delta H2)/(V1 + V2)$$

The SP value $\delta x$ of Mixture X is determined from the following equation.

$$\delta x = (\delta Dm^2 + \delta Pm^2 + \delta Hm^2)^{1/2}$$

In the composition of the present invention, even in a case where the component (A) having high crystallinity is used, crystallization of the component (A) in the coating film can be suppressed, the sustained release to the skin can be enhanced, and the storage stability of the composition can be improved. From the viewpoint of utility of this effect, the component (A) is preferably one or more crystalline medicinal ingredients selected from the group consisting of glycol salicylate, loxoprofen sodium, felbinac, l-menthol, and nonylic acid vanillylamide. However, in the composition of the present invention, even when the amorphous component (A) is used, a composition capable of forming a coating film having a high sustained release to the skin can be obtained.

<Component (B): Water-Insoluble Polymer>

The composition of the present invention contains a water-insoluble polymer as the component (B). It is considered that the component (B) is a film-forming component, improves the film-forming property and the quick-drying property of the composition, suppresses the stickiness of the coating film, and enables the formation of a coating film having high sustained release of the component (A) to the skin, appearance, and durability by the action mechanism described above.

From the viewpoint of improving the film-forming property, the component (B) is preferably a film-forming water-insoluble polymer. The definition of the "water-insoluble" of the component (B) is as described above.

The solubility parameter (SP value) of the component (B) is preferably 17.00 or more, more preferably 18.00 or more, still more preferably 19.00 or more, and even more preferably 20.00 or more, from the viewpoint of suppressing the tense feeling of the coating film to be formed, and from the viewpoint of improving the sustained release of the component (A) to the skin. On the other hand, from the viewpoint of suppressing the stickiness of the coating film to be formed, from the viewpoint of improving the sustained release of the component (A) to the skin, and from the viewpoint of improving the durability of the coating film, it is preferably 27.00 or less, more preferably 25.00 or less, still more preferably 24.00 or less, and even more preferably 23.00 or less. Thus, the SP value of the component (B) is preferably 17.00 or more and 27.00 or less, more preferably 18.00 or more and 25.00 or less, still more preferably 19.00 or more and 24.00 or less, and even more preferably 20.00 or more and 23.00 or less. The SP value can be calculated by the same method as described above.

The type of the component (B) is not particularly limited as long as it is a water-insoluble polymer, but from the viewpoint of suppressing the stickiness and the tense feeling of the coating film and from the viewpoint of improving the affinity to the skin, the film-forming property, and the durability, the component (B) is preferably one or more water-insoluble polymers selected from the group consisting of an acrylic polymer, a cellulosic polymer, and a vinyl polymer. More preferably, the component (B) is one or more water-insoluble polymers selected from the group consisting of an acrylic polymer, a cellulosic polymer, and a vinyl polymer, and has an SP value in the above range.

Examples of the acrylic polymer used as the component (B) include water-insoluble polymers among polymers containing at least a constituent unit derived from a monomer having a (meth)acrylic group. In the description herein, "(meth)acrylic" means both acrylic and methacrylic.

Specific examples of the acrylic polymer include water-insoluble polymers among (acrylates/diacetoneacrylamide) copolymer, acrylamide-methoxypolyethylene glycol methacrylate copolymer, acrylic acid-octyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammoniumethyl methacrylate chloride copolymer, methyl methacrylate-butyl methacrylate-dimethylaminoethyl methacrylate copolymer, and the like.

Among the above, as the (acrylate/diacetoneacrylamide) copolymer, the cosmetic ingredient label name "(alkyl acrylate/diacetoneacrylamide) copolymer AMP" can be used; as the acrylamide-methoxypolyethylene glycol methacrylate copolymer, "acrylamide-methoxypolyethylene glycol methacrylate copolymer solution" described in Japanese Pharmaceutical Excipients 2018 (Pharmaceutical Evaluation and Licensing Division, Pharmaceutical Safety and Environmental Health Bureau, Ministry of Health, Labor and Welfare) can be used; as the 2-ethylhexyl acrylate-vinylpyrrolidone copolymer, "2-ethylhexyl acrylate-vinylpyrrolidone copolymer solution" can be used; as the 2-ethylhexyl acrylate-2-ethylhexyl methacrylate-dodecyl methacrylate copolymer, "2-ethylhexyl acrylate-2-ethylhexyl methacrylate-dodecyl methacrylate copolymer solution" can be used; as the ethyl acrylate-methyl methacrylate copolymer, "ethyl acrylate-methyl methacrylate copolymer dispersion liquid" can be used; and as the methyl methacrylate-butyl methacrylate-dimethylaminoethyl methacrylate copolymer, "aminoalkyl methacrylate copolymer E" can be used.

In addition, as the ethyl acrylate-methyl methacrylate-trimethylammoniumethyl methacrylate chloride copolymer, "Aminoalkyl Methacrylate Copolymer RS" known as a pharmaceutical additive can be used.

Examples of the cellulosic polymer used as the component (B) include water-insoluble polymers among polymers having a cellulose skeleton.

Examples of the cellulosic polymer include water-insoluble polymers among methylcellulose, ethylcellulose, hypromellose, carboxymethylcellulose, hydroxyethylcellulose, hydroxypropylcellulose, hypromellose phthalate, hydrophobized (C 16-18) hydroxypropylmethylcellulose, and the like.

Examples of the vinyl polymer used as the component (B) include water-insoluble polymers among polymers which include at least a constituent unit derived from a monomer having a vinyl group and which do not belong to the acrylic polymers. Examples of the vinyl polymer include water-insoluble polymers among polyvinyl alcohol, polyvinyl butyral, and the like.

As the component (B), one kind or two or more kinds can be used.

Among the above, the component (B) is preferably one or more selected from the group consisting of (acrylates/diacetoneacrylamide) copolymer, acrylamide-methoxypolyethylene glycol methacrylate copolymer, acrylic acid-octyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammoniumethyl methacrylate chloride copolymer, methyl methacrylate-butyl methacrylate-dimethylaminoethyl methacrylate copolymer, methylcellulose, ethylcellulose, hypromellose, carboxymethylcellulose, hydroxyethylcellulose, hydroxypropylcellulose, hypromellose phthalate, hydrophobized (C16-18) hydroxypropylmethylcellulose, polyvinyl alcohol, and polyvinyl butyral, and more preferably one or more selected from the group consisting of (acrylates/diacetoneacrylamide) copolymer, acrylamide-methoxypolyethylene glycol methacrylate copolymer, ethylcellulose, hypromellose phthalate, and polyvinyl butyral, and has an SP value in the above range.

<Component (C): Non-volatile Base>

The composition of the present invention contains a non-volatile base (excluding those corresponding to the component (A)) as the component (C). It is considered that the component (C) improves the film-forming property, the coating property to the skin, and the quick-drying property of the composition by a synergistic effect with the component (B), and contributes to the sustained release of the component (A) to the skin and the improvement of the storage stability of the composition.

The definition of the "non-volatile base" of the component (C) is as described above.

Examples of the component (C) include a lipophilic base, a hydrophilic base, and an amphipathic base. From the viewpoint of improving the film-forming property, the coating property to the skin, and the quick-drying property of the composition, and from the viewpoint of enhancing the sustained release of the component (A) to the skin, one or more selected from the group consisting of a lipophilic base and a hydrophilic base are preferred.

In addition, from the viewpoint of the film-forming property and from the viewpoint of improving the storage stability of the composition, the component (C) is preferably a compound not containing an unsaturated hydrocarbon chain having 16 or more carbon atoms. In the component (C), the content of the non-volatile base containing an unsaturated hydrocarbon chain having 16 or more carbon atoms is preferably 30% by mass or less, more preferably 10% by mass or less, still more preferably 5% by mass or less, even more preferably 1% by mass or less, and even more preferably 0% by mass, from the viewpoint of the film-forming property and from the viewpoint of improving the storage stability of the composition.

Preferred examples of the lipophilic base include an ester oil and a nonpolar oil.

Specific examples of the ester oil include a non-volatile synthetic ester oil, and more preferred examples thereof include one or more selected from the group consisting of (i) a fatty acid monoester composed of a fatty acid and a monohydric alcohol, (ii) a fatty acid diester composed of a fatty acid and a dihydric alcohol, (iii) a dicarboxylic acid diester composed of a dicarboxylic acid and a monohydric alcohol, (iv) a tricarboxylic acid triester composed of a tricarboxylic acid and a monohydric alcohol, and (v) a glycerol fatty acid triester.

Examples of (i) the fatty acid monoester composed of a fatty acid and a monohydric alcohol include monoesters of a saturated fatty acid having 8 or more and 22 or less carbon atoms and an aliphatic or aromatic ring-containing monohydric alcohol having 1 or more and 24 or less carbon atoms, and examples thereof include cetyl octanoate, cetyl 2-ethylhexanoate, ethyl laurate, hexyl laurate, isopropyl myristate, myristyl myristate, hexadecyl myristate, 2-hexyldecyl myristate, octyldodecyl myristate, isopropyl palmitate, ethylhexyl palmitate, hexadecyl palmitate, 2-hexyldecyl palmitate, 2-heptylundecyl palmitate, butyl stearate, 2-ethylhexyl stearate, isocetyl stearate, isocetyl isostearate, isononyl isononanoate, and isotridecyl isononanoate.

Examples of (ii) the fatty acid diester composed of a fatty acid and a dihydric alcohol include diesters of a saturated fatty acid having 8 or more and 22 or less carbon atoms and an aliphatic or aromatic dihydric alcohol having 1 or more and 12 or less carbon atoms, and examples thereof include ethylene glycol di-2-ethylhexanoate, ethylene glycol dilaurate, ethylene glycol distearate, neopentyl glycol dicaprate, neopentyl glycol diethylhexanoate, propanediol di(caprylate/caprate), and propanediol diisostearate.

Examples of (iii) the dicarboxylic acid diester composed of a dicarboxylic acid and a monohydric alcohol include diesters of an aliphatic or aromatic dicarboxylic acid having 4 or more and 18 or less carbon atoms and an aliphatic or aromatic ring-containing monohydric alcohol having 1 or more and 24 or less carbon atoms, and examples thereof include 2-ethylhexyl succinate, dibutyl adipate, diisobutyl adipate, 2-hexyldecyl adipate, di-2-heptylundecyl adipate, diethyl sebacate, diisopropyl sebacate, di-2-ethylhexyl sebacate, and diisononyl phthalate.

Examples of (iv) the tricarboxylic acid triester composed of a tricarboxylic acid and a monohydric alcohol include triesters of an aliphatic or aromatic tricarboxylic acid having 5 or more and 12 or less carbon atoms and an aliphatic or aromatic ring-containing monohydric alcohol having 1 or more and 24 or less carbon atoms, and examples thereof include triisodecyl trimellitate.

Examples of (v) the glycerol fatty acid triester include triesters of glycerol and a saturated fatty acid having 8 or more and 22 or less carbon atoms, and examples thereof include glycerol tri-2-ethylhexanoate, glycerol trimyristate, glycerol tri-2-heptylundecanoate, and tristearin.

As the ester oil other than the above-mentioned (i) to (v), alkyl benzoate, hexyldecyl dimethyloctanoate, cetyl lactate, myristyl lactate, lanolin acetate, dipentaerythritol fatty acid ester, diisostearyl malate, glycerol di-2-heptylundecanoate, castor oil fatty acid methyl ester, N-lauroyl-L-glutamic acid-2-octyldodecyl, 2-hexyldecyl adipate, or the like can be used.

Among these, as the ester oil, one or more selected from the group consisting of (i) a fatty acid monoester composed of a fatty acid and a monohydric alcohol, and (iii) a dicarboxylic acid diester composed of a dicarboxylic acid and a monohydric alcohol are more preferable, one or more selected from the group consisting of a fatty acid monoester composed of a fatty acid and a monohydric alcohol having 1 or more and 24 or less carbon atoms, and a dicarboxylic acid diester composed of a dicarboxylic acid having 4 or more and 18 or less carbon atoms and a monohydric alcohol having 1 or more and 24 or less carbon atoms are still more preferable, and one or more selected from the group consisting of isopropyl myristate, dibutyl adipate, diethyl sebacate, and diisopropyl sebacate are even more preferable.

Specific examples of the nonpolar oil include a non-volatile hydrocarbon oil, a silicone oil, and a fluorine oil. Examples of the hydrocarbon oil include liquid paraffin and squalane; examples of the silicone oil include dimethylpolysiloxane, dimethylcyclopolysiloxane, methylphenylpolysiloxane, methylhydrogenpolysiloxane, and higher alcohol-modified organopolysiloxane; and examples of the fluorine oil include fluoropolyether and perfluoroalkyl ether silicone.

Examples of the hydrophilic base preferably include one or more selected from the group consisting of a polyol and a lower amine.

Specific examples of the polyol include non-volatile, alkylene glycols, polyalkylene glycols, and glycerols. Examples of the alkylene glycols include ethylene glycol, propylene glycol, 1,3-propanediol, 1,3-butylene glycol (1,3-butanediol), and 1,2-pentanediol; examples of the polyalkylene glycols include diethylene glycol, dipropylene glycol, polyethylene glycol, polypropylene glycol, and polyoxyethylene-polyoxypropylene glycol; and examples of the glycerols include glycerol, diglycerol, and triglycerol. The polyethylene glycol, polypropylene glycol, and polyoxyethylene-polyoxypropylene glycol preferably have a weight average molecular weight of 10,000 or less.

In the description herein, the term "lower amine" refers to an amine having preferably 9 or less carbon atoms, and more preferably 2 or more and 9 or less carbon atoms. The lower amine is preferably an alkanolamine from the viewpoint of non-volatility, from the viewpoint of improving the film-forming property, the coating property to the skin, and the quick-drying property of the composition, and from the viewpoint of enhancing the sustained release of the component (A) to the skin.

Specific examples of the alkanolamine include triethanolamine, diethanolamine, monoethanolamine, triisopropanolamine, diisopropanolamine, monoisopropanolamine, and 2-amino-2-methylpropanol.

As the component (C), one kind or two or more kinds can be used.

Among the above, the component (C) is preferably one or more selected from the group consisting of ester oils, nonpolar oils, polyols, and alkanolamines, more preferably one or more selected from the group consisting of isopropyl myristate, dibutyl adipate, diethyl sebacate, diisopropyl sebacate, propylene glycol, 1,3-butylene glycol, 1,2-pentanediol, dipropylene glycol, polyethylene glycol, polypropylene glycol, polyoxyethylene/polyoxypropylene glycol, glycerol, diglycerol, triethanolamine, monoethanolamine, diisopropanolamine, and 2-amino-2-methylpropanol, and still more preferably one or more selected from the group consisting of isopropyl myristate, diethyl sebacate, triethanolamine, diisopropanolamine, and 2-amino-2-methylpropanol.

<Component (D): Volatile Solvent>

The composition of the present invention contains a volatile solvent (excluding those corresponding to the component (A)) as the component (D). The component (D) contributes to the improvement of the coating property, quick-drying property, storage stability, and the like of the composition.

The definition of the "volatile solvent" of the component (D) is as described above.

Examples of the component (D) include an alcohol, a ketone, an ester, a hydrocarbon, a silicone, and the like having volatility.

The alcohol used as the component (D) is preferably a lower alcohol. The "lower alcohol" is preferably a monohydric alcohol having 4 or less carbon atoms, and examples thereof include methanol, ethanol, n-propyl alcohol, isopropyl alcohol, n-butyl alcohol, isobutyl alcohol, sec-butyl alcohol, and tert-butyl alcohol.

Examples of the ketone used as the component (D) include acetone, ethyl methyl ketone, and methyl isobutyl ketone; examples of the ester include methyl acetate, ethyl acetate, and butyl acetate; examples of the hydrocarbon include volatile liquid paraffin; and examples of the silicone include linear polydimethylsiloxane and cyclic siloxane.

As the component (D), one kind or two or more kinds can be used.

From the viewpoint of improving the coating property and quick-drying property of the composition, from the viewpoint of the solubility or dispersibility of the components (A) to (C), and from the viewpoint of the storage stability of the composition, the component (D) is preferably a lower alcohol, and more preferably one or more kinds selected from the group consisting of ethanol and isopropyl alcohol.

<Component (E): Water>

The composition of the present invention contains water as the component (E). The component (E) contributes to the improvement of the storage stability, and the like.

The component (E) is not particularly limited, and for example, ion-exchanged water, pure water, distilled water, or the like can be used.

<Other Components>

The composition for external application of the present invention may appropriately contain, in addition to the above-mentioned components, other components which are usually blended in composition for external application within a range not impairing the object of the present invention. Examples of the component include a surfactant, a water-soluble polymer, an antioxidant, an ultraviolet absorber, a vitamin tablet, an antiseptic, a pH adjuster, a fragrance, a plant extract other than the component (A), a humectant, a colorant, a refreshing agent, an antiperspirant, and a skin activator.

<Content>

The content of each of the components in the composition for external application of the present invention is preferably as follows from the viewpoint of obtaining a composition for external application which can sustainedly release a medicinal ingredient to the skin in a sustained manner, which has good film-forming property, high coating property to the skin, and high quick-drying property, and is excellent in the effects of suppressing stickiness and tense feeling of a coating film to be formed, and improving the appearance, durability, and the like.

The content of the component (A) in the composition is preferably 0.001% by mass or more, more preferably 0.005% by mass or more, still more preferably 0.01% by mass or more, even more preferably 0.1% by mass or more, and even more preferably 1.0% by mass or more from the viewpoint of securing a sufficient effect as a medicinal ingredient, and is preferably 30% by mass or less, more preferably 20% by mass or less, still more preferably 15% by mass or less, even more preferably 10% by mass or less, and even more preferably 5.0% by mass or less from the viewpoint of improving the storage stability of the composition. Thus, the content of the component (A) in the composition is preferably 0.001% by mass or more and 30% by mass or less, more preferably 0.005% by mass or more and 20% by mass or less, still more preferably 0.01% by mass or more and 15% by mass or less, still more preferably 0.1% by mass or more and 10% by mass or less, and still more preferably 1.0% by mass or more and 5.0% by mass or less.

In a case where the component (A) contains glycol salicylate, the content of glycol salicylate in the composition is preferably 0.1% by mass or more and 30% by mass or less, more preferably 0.5% by mass or more and 20% by mass or less, still more preferably 1.0% by mass or more and 15% by mass or less, still more preferably 2.0% by mass or more and 12% by mass or less, and still more preferably 2.0% by mass or more and 5.0% by mass or less. In a case where the component (A) contains sodium loxoprofen, the content of sodium loxoprofen in the composition is preferably 0.01% by mass or more and 20% by mass or less, more preferably 0.05% by mass or more and 10% by mass or less, and still more preferably 0.1% by mass or more and 5.0% by mass or less. In a case where the component (A) contains felbinac, the content of felbinac in the composition is preferably 0.01% by mass or more and 30% by mass or less, more preferably 0.1% by mass or more and 20% by mass or less, still more preferably 0.5% by mass or more and 15% by mass or less, even more preferably 1.0% by mass or more and 10% by mass or less, and even more preferably 1.5% by mass or more and 5.0% by mass or less. In a case where the component (A) contains l-menthol, the content of l-menthol in the composition is preferably 0.01% by mass or more and 30% by mass or less, more preferably 0.1% by mass or more and 20% by mass or less, and still more preferably 0.5% by mass or more and 10% by mass or less. In a case where the component (A) contains nonylic acid vanillylamide, the content of nonylic acid vanillylamide in the composition is preferably 0.001% by mass or more and 15% by mass or less, more preferably 0.005% by mass or more and 10% by mass or less, still more preferably 0.01% by mass or more and 5.0% by mass or less, still more preferably 0.01% by mass or more and 1.0% by mass or less, and still more preferably 0.01% by mass or more and 0.1% by mass or less.

The content of the component (B) in the composition is preferably 0.01% by mass or more, more preferably 0.05% by mass or more, still more preferably 0.1% by mass or more, even more preferably 0.5% by mass or more, and even more preferably 1.0% by mass or more from the viewpoint of the film-forming property and the durability of the coating film, and is preferably 30% by mass or less, more preferably 25% by mass or less, still more preferably 20% by mass or less, even more preferably 12% by mass or less, and even more preferably 5.0% by mass or less from the viewpoint of improving the coating property and the quick-drying property of the composition. Thus, the content of the component (B) in the composition is preferably 0.01% by mass or more and 30% by mass or less, more preferably 0.05% by mass or more and 25% by mass or less, still more preferably 0.1% by mass or more and 20% by mass or less, even more preferably 0.5% by mass or more and 12% by mass or less, and even more preferably 1.0% by mass or more and 5.0% by mass or less.

In a case where the component (B) contains (acrylates/diacetoneacrylamide) copolymer, the content of the copolymer in the composition is even more preferably 0.1% by mass or more and 15% by mass or less, even more preferably 0.5% by mass or more and 10% by mass or less, and even more preferably 1.5% by mass or more and 5.0% by mass or less. In a case where the component (B) contains acrylamide-methoxypolyethylene glycol methacrylate copolymer, the content of the copolymer in the composition is even more preferably 0.1% by mass or more and 10% by mass or less. In a case where the component (B) contains ethylcellulose, the content of ethylcellulose in the composition is even more preferably 0.1% by mass or more and 20% by mass or less. In a case where the component (B) contains hypromellose phthalate, the content of hypromellose phthalate in the composition is even more preferably 0.1% by mass or more and 20% by mass or less, even more preferably 0.5% by mass or more and 15% by mass or less, even more preferably 1.0% by mass or more and 10% by mass or less, and even more preferably 1.5% by mass or more and 5.0% by mass or less. In a case where the component (B) contains polyvinyl butyral, the content of polyvinyl butyral in the composition is even more preferably 0.1% by mass or more and 20% by mass or less.

The content of the component (C) in the composition is preferably 0.001% by mass or more, more preferably 0.005% by mass or more, still more preferably 0.01% by mass or more, still more preferably 0.1% by mass or more, and still more preferably 1.0% by mass or more from the viewpoint of improving the sustained release of the component (A) to the skin and suppressing the tense feeling of the coating film to be formed, and is preferably 30% by mass or less, more preferably 20% by mass or less, still more preferably 10% by mass or less, even more preferably 8.0% by mass or less, and even more preferably 5.0% by mass or less from the viewpoint of suppressing the stickiness of the coating film to be formed and from the viewpoint of improving the rubfastness. Thus, the content of the component (C) in the composition is preferably 0.001% by mass or more and 30% by mass or less, more preferably 0.005% by mass or more and 20% by mass or less, even more preferably 0.1% by mass or more and 15% by mass or less, even more preferably 0.01% by mass or more and 10% by mass or less, even more preferably 0.1% by mass or more and 8.0% by mass or less, and even more preferably 1.0% by mass or more and 5.0% by mass or less.

In a case where the component (C) contains isopropyl myristate, the content of isopropyl myristate in the composition is even more preferably 0.1% by mass or more and 10% by mass or less, even more preferably 0.25% by mass or more and 7.5% by mass or less, and even more preferably 0.5% by mass or more and 5.0% by mass or less. In a case where the component (C) contains diethyl sebacate, the content of diethyl sebacate in the composition is even more preferably 0.1% by mass or more and 10% by mass or less, even more preferably 0.25% by mass or more and 7.5% by mass or less, and even more preferably 0.5% by mass or more and 5.0% by mass or less. In a case where the component (C) contains diisopropanolamine, the content of diisopropanolamine in the composition is even more preferably 0.1% by mass or more and 5.0% by mass or less, even more preferably 0.5% by mass or more and 4.0% by mass or less, and even more preferably 1.0% by mass or more and 3.0% by mass or less. In a case where the component (C) contains triethanolamine, the content of triethanolamine in the composition is even more preferably 0.1% by mass or more and 5.0% by mass or less, and even more preferably 1.0% by mass or more and 3.0% by mass or less. In a case where the component (C) contains 2-amino-2-methylpropanol, the ontent of 2-amino-2-methylpropanol in the composition is even more preferably 0.01% by mass or more and 3.0% by mass or less.

The content of the component (D) in the composition is preferably 10% by mass or more, more preferably 25% by mass or more, still more preferably 45% by mass or more, and even more preferably 65% by mass or more from the viewpoint of improving the coating property, the quick-drying property, and the storage stability of the composition, and is preferably 95% by mass or less, more preferably 90% by mass or less, still more preferably 80% by mass or less, and even more preferably 75% by mass or less from the viewpoint of maintaining the coating property and the film-forming property of the composition. Thus, the content of the component (D) in the composition is preferably 10% by mass or more and 95% by mass or less, more preferably 25% by mass or more and 90% by mass or less, still more preferably 45% by mass or more and 80% by mass or less, and even more preferably 65% by mass or more and 75% by mass or less.

In a case where ethanol is contained as the component (D), the content of ethanol in the composition is even more preferably 30% by mass or more and 90% by mass or less, even more preferably 30% by mass or more and 75% by mass or less, and even more preferably 45% by mass or more and 75% by mass or less. In a case where isopropyl alcohol is contained as the component (D), the content of isopropyl alcohol in the composition is even more preferably 30% by mass or more and 90% by mass or less, even more preferably 30% by mass or more and 75% by mass or less, and even more preferably 45% by mass or more and 75% by mass or less.

In addition, the content of the component (E) in the composition is preferably 0.1% by mass or more, more preferably 0.5% by mass or more, still more preferably 5% by mass or more, and even more preferably 13% by mass or more from the viewpoint of improving the coating property and the storage stability of the composition and from the viewpoint of maintaining the film-forming property, and is preferably 70% by mass or less, more preferably 50% by mass or less, still more preferably 30% by mass or less, even more preferably 25% by mass or less, even more preferably 22% by mass or less, even more preferably 21% by mass or less, and even more preferably 20% by mass or less from the viewpoint of the coating property of the composition, from the viewpoint of maintaining the quick-drying property of the composition, from the viewpoint of improving the storage stability, and from the viewpoint of maintaining a good appearance during low-temperature storage. Thus, the content of the component (E) in the composition is preferably 0.1% by mass or more and 70% by mass or less, more preferably 0.5% by mass or more and 50% by mass or less, still more preferably 0.5% by mass or more and 30% by mass or less, even more preferably 0.5% by mass or more and 25% by mass or less, even more preferably 0.5% by mass or more and 22% by mass or less, even more preferably 5% by mass or more and 22% by mass or less, even more preferably 5% by mass or more and 21% by mass or less, and even more preferably 13% by mass and 20% by mass or less.

The content of the component (E) in the composition can be set to more than 30% by mass and 70% by mass or less from the viewpoint of improving the coating property and the storage stability of the composition, from the viewpoint of maintaining the film-forming property, and from the viewpoint of maintaining a good appearance during low-temperature storage.

The total content of the components (A) to (E) in the composition is preferably 50% by mass or more, more preferably 70% by mass or more, still more preferably 80% by mass or more, and even more preferably 90% by mass or more, and on the other hand, it is 100% by mass or less, from the viewpoint of obtaining the effects of the present invention.

The mass ratio [(A)/(B)] of the component (A) to the component (B) in the composition is preferably 0.01 or more, more preferably 0.02 or more, still more preferably 0.03 or more, even more preferably 0.05 or more, even more preferably 0.10 or more, and even more preferably 0.15 or more from the viewpoint of improving the sustained release of the component (A) to the skin, and is preferably 50 or less, more preferably 30 or less, still more preferably 20 or less, even more preferably 10 or less, even more preferably 5.0 or less, and even more preferably 3.0 or less from the viewpoint of improving the coating property, the storage stability, and the film-forming property of the composition, and the film-forming property and the durability of the coating film. Thus, the mass ratio [(A)/(B)] in the composition is preferably 0.01 or more and 50 or less, more preferably 0.02 or more and 30 or less, still more preferably 0.03 or more and 20 or less, even more preferably 0.05 or more and 10 or less, even more preferably 0.10 or more and 5.0 or less, and even more preferably 0.15 or more and 3.0 or less.

The mass ratio [(A)/(C)] of the component (A) to the component (C) in the composition is preferably 0.01 or more, more preferably 0.02 or more, still more preferably 0.03 or more, even more preferably 0.05 or more, even more preferably 0.10 or more, and even more preferably 0.15 or more from the viewpoint of improving the sustained release of the component (A) to the skin and from the viewpoint of improving the storage stability of the composition, and is preferably 10 or less, more preferably 5.0 or less, still more preferably 3.0 or less, even more preferably 2.5 or less, even more preferably 2.0 or less, and even more preferably 1.5 or less from the viewpoint of improving the coating property and the storage stability of the composition, and from the viewpoint of improving the durability of the coating film to be formed. Thus, the mass ratio [(A)/(C)] in the composition is preferably 0.01 or more and 10 or less, more preferably 0.02 or more and 5.0 or less, still more preferably 0.03 or more and 3.0 or less, even more preferably 0.05 or more and 2.5 or less, even more preferably 0.10 or more and 2.0 or less, and even more preferably 0.15 or more and 1.5 or less.

The mass ratio [(C)/(B)] of the component (C) to the component (B) in the composition is preferably 0.05 or more, more preferably 0.10 or more, still more preferably 0.20 or more, and even more preferably 0.50 or more from the viewpoint of improving the sustained release of the component (A) to the skin and from the viewpoint of improving the film-forming property, and is preferably 50 or less, more preferably 35 or less, still more preferably 20 or less, even more preferably 10 or less, even more preferably 5.0 or less, and even more preferably 3.5 or less from the viewpoint of the durability of the coating film to be formed. Thus, the mass ratio [(C)/(B)] in the composition is preferably 0.05 or more and 50 or less, more preferably 0.10 or more and 35 or less, still more preferably 0.20 or more and 20 or less, even more preferably 0.20 or more and 10 or less, even more preferably 0.20 or more and 5.0 or less, even more preferably 0.50 or more and 5.0 or less, and even more preferably 0.50 or more and 3.5 or less. Alternatively, the mass ratio [(C)/(B)] in the composition is preferably 0.05 or more and 50 or less, more preferably 0.05 or more and 35 or less, still more preferably 0.05 or more and 20 or less, even more preferably 0.05 or more and 10 or less, even more preferably 0.05 or more and 5.0 or less, even more preferably 0.05 or more and 5.0 or less, and even more preferably 0.05 or more and 3.5 or less.

The mass ratio [(E)/(D)] of the component (E) to the component (D) in the composition is preferably 0.005 or more, more preferably 0.05 or more, still more preferably 0.10 or more, even more preferably 0.16 or more, and even more preferably 0.20 or more from the viewpoint of improving the coating property, the quick-drying property and the storage stability of the composition, and is preferably 3.0 or less, more preferably 2.0 or less, still more preferably 1.0 or less, even more preferably 0.80 or less, and even more preferably 0.40 or less from the same viewpoint. Thus, the mass ratio [(E)/(D)] in the composition is preferably 0.005 or more and 3.0 or less, more preferably 0.05 or more and 2.0 or less, still more preferably 0.10 or more and 1.0 or less, even more preferably 0.16 or more and 0.80 or less, and even more preferably 0.20 or more and 0.40 or less.

The mass ratio [(B)/{(D) + (E)}] of the component (B) to the total content of the component (D) and the component (E) in the composition is preferably 0.001 or more, more preferably 0.01 or more, and still more preferably 0.02 or more from the viewpoint of improving the film-forming property, and is preferably 1.0 or less, more preferably 0.50 or less, still more preferably 0.20 or less, even more preferably 0.12 or less, and even more preferably 0.10 or less from the viewpoint of improving the coating property and the quick-drying property. Thus, the mass ratio [(B)/{(D) + (E)}] in the composition is preferably 0.001 or more and 1.0 or less, more preferably 0.01 or more and 0.50 or less, still more preferably 0.01 or more and 0.20 or less, even more preferably 0.01 or more and 0.12 or less, and even more preferably 0.02 or more and 0.10 or less.

The mass ratio [(A)/{(B) + (C)}] of the component (A) to the total amount of the component (B) and the component (C) in the composition is preferably 0.001 or more, more preferably 0.005 or more, still more preferably 0.01 or more, even more preferably 0.05 or more, and even more preferably 0.10 or more from the viewpoint of ensuring sufficient effect as a medicinal ingredient and from the viewpoint of improving the sustained release of the component (A) to the skin, and is preferably 5.00 or less, more preferably 3.00 or less, still more preferably 2.50 or less, even more preferably 2.40 or less, even more preferably 2.00 or less, even more preferably 1.60 or less, even more preferably 1.00 or less, and even more preferably 0.60 or less from the viewpoint of suppressing the crystallization of the component (A), from the viewpoint of improving the durability of the coating film to be formed, and from the viewpoint of improving the storage stability of the composition. Thus, the mass ratio [(A)/{(B) + (C)}] in the composition is preferably 0.001 or more and 5.00 or less, more preferably 0.005 or more and 3.00 or less, still more preferably 0.01 or more and 2.50 or less, even more preferably 0.05 or more and 2.50 or less, even more preferably 0.10 or more and 2.50 or less, even more preferably 0.10 or more and 1.60 or less, even more preferably 0.10 or more and 1.00 or less, and even more preferably 0.10 or more and 0.60 or less. In particular, from the viewpoint of suppressing the crystallization of the component (A), it is preferably 0.001 or more and 2.40 or less, more preferably 0.005 or more and 2.00 or less, still more preferably 0.01 or more and 1.60 or less, even more preferably 0.05 or more and 1.00 or less, and even more preferably 0.05 or more and 0.60 or less.

<Viscosity>

The viscosity of the composition for external application of the present invention at 25°C is 1.0 mPa s or more, preferably 3.0 mPa s or more, more preferably 5.0 mPa s or more, and still more preferably 10.0 mPa ·s or more from the viewpoint of improving the coating property to the skin and the evenness of the coating film to be formed. On the other hand, from the viewpoint of improving the quick-drying property of the composition and from the viewpoint of improving the appearance of the coating film to be formed, the viscosity is 10,000 mPa ·s or less, preferably 7,500 mPa s or less, more preferably 5,000 mPa s or less, still more preferably 3,000 mPa ·s or less, even more preferably 2,000 mPa ·s or less, even more preferably 1,000 mPa s or less, and even more preferably 700 mPa ·s or less. Thus, the viscosity of the composition for external application at 25°C is 1.0 mPa ·s or more and 10,000 mPa s or less, preferably 3.0 mPa s or more and 7,500 mPa ·s or less, more preferably 3.0 mPa s or more and 5,000 mPa s or less, still more preferably 3.0 mPa ·s or more and 3,000 mPa ·s or less, even more preferably 3.0 mPa s or more and 2,000 mPa s or less, even more preferably 5.0 mPa ·s or more and 1,000 mPa s or less, and even more preferably 10.0 mPa s or more and 700 mPa ·s or less.

The viscosity of the composition for external application at 25°C is a value measured by a vibration-type viscometer for a composition having a viscosity of 1,000 mPa ·s or less and by a B-type rotational viscometer for a composition having a viscosity of more than 1,000 mPa ·s, and specifically, the viscosity can be measured by the method described in Examples.

<$\Delta(SP_A - SP_{B+C})$>

In the composition of the present invention, the absolute value [$\Delta(SP_A - SP_{B+C})$] of the difference between the SP value ($SP_A$) of the component (A) and the SP value ($SP_{B+C}$) of the mixture of the component (B) and the component (C) is preferably 0.10 or more, more preferably 0.15 or more, still more preferably 0.20 or more, even more preferably 0.30 or more, and even more preferably 0.50 or more, and is preferably 10.00 or less, more preferably 8.00 or less, still more preferably 7.00 or less, and even more preferably 5.00 or less, from the viewpoint of improving the sustained release of the component (A) to the skin and from the viewpoint of improving the storage stability of the composition. Thus, $[\Delta(SP_A\text{-}SP_{B+C})]$ is preferably 0.10 or more and 10.00 or less, more preferably 0.15 or more and 8.00 or less, still more preferably 0.20 or more and 7.00 or less, even more preferably 0.30 or more and 5.00 or less, and even more preferably 0.50 or more and 5.00 or less.

When $[\Delta(SP_A\text{-}SP_{B+C})]$ is 0.10 or more, the compatibility between the component (A) and the coating film does not become excessively high, the spinnability of the coating film to be formed can be suppressed, and the sustained release of the component (A) to the skin side is favorably maintained. On the other hand, when $[\Delta(SP_A\text{-}SP_{B+C})]$ is 10.00 or less, the compatibility between the component (A) and the coating film does not become excessively low, and the crystallization of the crystalline component (A) can be suppressed, so that the sustained release of the component (A) to the skin side is favorably maintained.

The SP value ($SP_A$) of the component (A) and the SP value ($SP_{B+C}$) of the mixture of the component (B) and the component (C) can be calculated by the same method as described above.

<Dosage form, etc.>

The composition for external application of the present invention is preferably used as a composition for skin external application.

The dosage form of the composition for external application of the present invention may be in any form as long as it has a viscosity within the above range and can be applied to the skin. Examples thereof include a lotion formulation, a gel formulation, an ointment formulation, a cream formulation, or a foam formulation made of the composition for external application of the present invention; and an aerosol formulation or a pump spray type formulation using the composition for external application of the present invention. Examples of the foam formulation include formulations used by filling a pump foamer or the like with the composition for external application of the present invention and ejecting it in a foam form.

The compositions of the present invention can also be electrostatically sprayed onto the skin using an electrostatic spray device. The electrostatic spraying will be described later.

The aerosol formulation contains the composition for external application and a propellant. The aerosol formulation using the composition for external application of the present invention can provide cool feeling and the like by spraying and applying the composition to the skin.

In the aerosol formulation, the composition for external application used as an aerosol stock solution and the suitable aspect thereof are the same as described above. That is, the content of the components (A) to (E) in the aerosol stock solution and the viscosity are preferably in the ranges described above.

Examples of the propellant used in the aerosol formulation include liquefied petroleum gas (LPG) which is ethane, propane, normal butane, isobutane, isopentane, and a mixture thereof; ethers such as dimethyl ether; and compressed gases such as nitrogen and carbon dioxide, and one or more of these can be used.

In the aerosol formulation, the quantitative ratio between the composition for external application which is the aerosol stock solution and the propellant is not particularly limited as long as the composition for external application can be sprayed to the skin, but from the viewpoint of aerosol performances and the stabilities of the component (A), the mass ratio of the composition for external application to the propellant is preferably in the range of 1:0.01 to 1:10, and more preferably in the range of 1:0.05 to 1:7.5.

When the propellant is LPG, the mass ratio of the composition for external application to LPG is still more preferably 1:0.5 to 1:5, when the propellant is dimethyl ether, the mass ratio of the composition for external application to dimethyl ether is still more preferably 1:0.1 to 1:5, and when the propellant is carbon dioxide, the mass ratio of the composition for external application to carbon dioxide is still more preferably 1:0.1 to 1:0.5.

Examples of an aerosol container used in the aerosol formulation include a known pressure-resistant container made of metal, plastic, or the like, and a double-structured container in which an inner bag is accommodated inside the pressure-resistant container. In the double-structured container, it is preferable that the inner bag is filled with a composition for external application as an aerosol stock solution, and a propellant is filled between the pressure-resistant container and the inner bag.

The method for preparing the aerosol formulation is not particularly limited. For example, the aerosol formulation can be prepared by filling the aerosol container with a composition for external application which is an aerosol stock solution, attaching a valve to the container, and then filling a propellant from the valve portion.

In addition, the pump spray type formulation is a formulation used by filling the composition for external application of the present invention into a pump spray container.

[Method of Use]

The present invention also provides a method of using a composition for external application, which includes a step of applying the above-described composition for external application to the skin and then drying the composition.

According to the method of the present invention, a coating film containing the component (A) which is a medicinal ingredient can be formed to the skin surface by applying the composition for external application to the skin and then drying. The coating film has excellent moisture resistance and rubfastness, and has high sustained release of the component (A) to the skin, so that the component (A) can be sustainedly released into the skin in a sustained manner. Hereinafter, the step of applying the composition for external application to the skin and then drying it is also referred to as a "film forming step".

The method for applying the composition for external application to the skin can be appropriately selected according to the dosage form of the composition for external application, and examples thereof include methods for applying the composition by coating, casting, spraying, or the like. Among these, it is preferable to apply the composition by coating or spraying. After the application of the composition for external application, the composition is dried by natural drying or the like, whereby the component (D) is volatilized and a coating film containing the components (A) to (C) is formed on the skin surface.

In the method of the present invention, in a case where the composition for external application is directly applied to the skin, the composition for external application can be applied by hand or by using an applicator such as a metallic roller, a plastic roller, a sponge-like porous body, or a brush.

In the method of the present invention, in a case where the composition for external application is applied to the skin by spraying, the method for spraying the composition includes a method using an aerosol, a pump spray, or an electrostatic spray device. With respect to the aerosol and the pump spray, the aerosol formulation or the pump spray formulation is prepared using the composition for external application as a stock solution, and the composition for external application can be sprayed to the skin using the formulation.

The electrostatic spray device includes, specifically, a storage portion capable of storing the composition for external application, a nozzle for ejecting the composition for external application, a power source for applying a voltage to the nozzle, and a means for feeding the composition for external application from the storage portion to the nozzle. As one embodiment thereof, there is a handy type electrostatic spray device having a size which can be held by one hand.

As the electrostatic spray device, those exemplified in WO 2018/194140 and the like can be used.

The application site of the composition for external application is not particularly limited, but is preferably a skin surface of a body part, and more preferably a skin surface of a hand, an arm, a leg, a leg portion, or a trunk portion.

The amount of the composition for external application applied to the skin is not particularly limited, but is preferably 0.5 $\mu$L/cm$^2$ or more, more preferably 1 $\mu$L/cm$^2$ or more, and still more preferably 3 $\mu$L/cm$^2$ or more from the viewpoint of sufficiently sustainedly releasing the component (A) to the skin. On the other hand, from the viewpoint of the appearance (transparency) of the coating film to be formed, the amount is preferably 100 $\mu$L/cm$^2$ or less, and more preferably 50 $\mu$L/cm$^2$ or less.

[Method for Producing Coating Film]

The present invention also provides a method for producing a coating film, which includes a step of applying the composition for external application to the skin by direct application or spraying.

In a case where the composition for external application is applied to the skin by direct application, the composition for external application can be applied by hand or by using an applicator such as a metallic roller, as described above.

In a case where the composition for external application is applied to the skin by direct spraying, examples of the method for spraying the composition include a method using the aerosol, pump spray, or electrostatic spray device.

An embodiment of a method for producing a coating film in a case of applying the composition for external application to the skin by spraying the composition for external application by an electrostatic spray method using the composition for external application and an electrostatic spray device will be described below.

First, the storage portion of the handy type electrostatic spray device is filled with the composition for external application, and a user, that is, a person who forms a coating film on an application site of the skin by electrostatic spraying grasps the device by hand, and one end of the device in which a nozzle is disposed is directed toward an application site for performing electrostatic spraying.

Under this condition, the electrostatic spray device can be switched on to perform an electrostatic spray method. When the device is powered on, an electric field is generated between the nozzle and the skin, a positive high voltage is applied to the nozzle, and the skin serves as a negative electrode. When an electric field is generated between the nozzle and the skin, the composition for external application of the nozzle tip end portion is polarized by electrostatic induction so that the tip end portion becomes a cone shape, and droplets of the composition for external application charged from the cone tip end are ejected into the air toward the skin along the electric field. When the volatile solvent which is the component (D) is evaporated from the composition for external application which is ejected into a space and charged, the charge density on the composition for external application surfaces becomes excessive, and the composition spreads in the space while repeating micronization by Coulomb repulsion, and reaches the skin. In this case, by appropriately adjusting the viscosity of the composition for external application, the amount of the volatile solvent, the type and amount of the polymer, the flow rate, the voltage, and the like, the sprayed composition can be made to reach the application site in the form of droplets. Preferably, the component (D) is volatilized from the droplet while the droplet is ejected into the space, and the component (B) which is a main film-forming component is solidified, and at the same time, a fiber is formed while the fiber is elongated and deformed by a potential difference, and the fiber can be deposited on the application site. A porous coating film composed of a deposit of fibers is thereby formed on the surface of the application site.

With respect to the embodiments described above, the present invention further discloses the following embodiments.

<1> A composition for external application, containing (A) a medicinal ingredient, (B) a water-insoluble polymer, (C) a non-volatile base, (D) a volatile solvent, and (E) water, in which the composition has a viscosity at 25°C of 1.0 mPa s or more, preferably 3.0 mPa ·s or more, more preferably 5.0 mPa ·s or more, and still more preferably 10.0 mPa s or more, and is 10,000 mPa s or less, preferably 7,500 mPa s or less, more preferably 5,000 mPa s or less, still more preferably 3,000 mPa ·s or less, even more preferably 2,000 mPa ·s or less, even more preferably 1,000 mPa ·s or less, and even more preferably 700 mPa ·s or less.

<2> A composition for external application, containing (A) a medicinal ingredient, (B) a water-insoluble polymer, (C) a non-volatile base, (D) a volatile solvent, and (E) water, in which the content of the component (E) in the composition for external application is 0.1% by mass or more and 30% by mass or less, the mass ratio [(A)/{(B) + (C)}] of the component (A) to the total amount of the component (B) and the component (C) is 0.001 or more and 2.40 or less, and the viscosity at 25°C is 1.0 mPa ·s or more and 10,000 mPa ·s or less.

<3> A composition for external application, containing (A) a medicinal ingredient, (B) a water-insoluble polymer, (C) a non-volatile base, (D) a volatile solvent, and (E) water, in which the content of the component (E) in the composition for external application is more than 30% by mass and 70% by mass or less, the mass ratio [(A)/{(B) + (C)}] of the component (A) to the total amount of the component (B) and the component (C) is 0.001 or more and 2.40 or less, and the viscosity at 25°C is 1.0 mPa ·s or more and 10,000 mPa ·s or less.

<4> A composition for external application, containing (A) an anti-inflammatory analgesic component, (B) a water-insoluble polymer, (C) a non-volatile base, (D) a volatile solvent, and (E) water, in which the content of the component (B) in the composition for external application is 0.1% by mass or more and 15% by mass or less, the mass ratio [(C)/(B)] of the component (C) to the component (B) in the composition for external application is 0.05 or more and 3.5 or less, and the viscosity at 25°C is 1.0 mPa s or more and 10,000 mPa ·s or less.

<5> A composition for external application, containing (A) one or more medicinal ingredients selected from the group consisting of glycol salicylate, diclofenac sodium, loxoprofen sodium, felbinac, indomethacin, piroxicam, flurbiprofen, methyl salicylate, ketoprofen, and nonylic acid vanillylamide, (B) a water-insoluble polymer, (C) a non-volatile base, (D) a volatile solvent, and (E) water, in which the content of the component (C) in the composition for external application is 5% by mass or less, and the viscosity at 25°C is 1.0 mPa -s or more and 10,000 mPa ·s or less.

<6> The composition for external application according to any one of <2> to <5>, in which the viscosity at 25°C of the composition for external application is preferably 3.0 mPa s or more, more preferably 5.0 mPa s or more, and still more preferably 10.0 mPa ·s or more, and is preferably 7,500 mPa -s or less, more preferably 5,000 mPa s or less, still more preferably 3,000 mPa ·s or less, even more preferably 2,000 mPa ·s or less, even more preferably 1,000 mPa ·s or less, and even more preferably 700 mPa ·s or less.

<7> The composition for external application according to any one of <1> to <6>, in which the SP value of the component (B) is preferably 17.00 or more, more preferably 18.00 or more, still more preferably 19.00 or more, and even more preferably 20.00 or more, and is preferably 27.00 or less, more preferably 25.00 or less, still more preferably 24.00 or less, and even more preferably 23.00 or less.

<8> The composition for external application according to any one of <1> to <7>, in which the absolute value [$\Delta(SP_A - SP_{B+C})$] of the difference between the SP value ($SP_A$) of the component (A) and the SP value ($SP_{B+C}$) of the mixture of the component (B) and the component (C) is preferably 0.10 or more, more preferably 0.15 or more, still more preferably 0.20 or more, even more preferably 0.30 or more, and even more preferably 0.50 or more, and is preferably 10.00 or less, more preferably 8.00 or less, still more preferably 7.00 or less, and even more preferably 5.00 or less.

<9> The composition for external application according to any one of <1> to <3> and <6> to <8>, in which the component (A) contains one or more selected from the group consisting of an anti-inflammatory analgesic component, a local irritation component, a blood circulation promoting component, an antihistamine component, a crude drug component, an antipruritic component, a local anesthetic component, a keratin softening component, a bactericidal component, and an antibacterial component, preferably contains one or more selected from the group consisting of an anti-inflammatory analgesic component, a local irritation component, a blood circulation promoting component, an antihistamine component, and a crude drug component, and more preferably contains one or more selected from the group consisting of an anti-inflammatory analgesic component and a local irritation component.

<10> The composition for external application according to any one of <1> to <3> and <6> to <9>, in which the component (A) contains one or more selected from the group consisting of glycol salicylate, methyl salicylate, diclofenac sodium, loxoprofen sodium, ketoprofen, felbinac, indomethacin, piroxicam, flurbiprofen, glycyrrhizic acid, glycyrrhetinic acid, l-menthol, dl-camphor, nonylic acid vanillylamide, peppermint oil, benzyl nicotinate, sodium polyethylene sulfonate, tocopherol acetate, diphenhydramine, chlorpheniramine maleate, capsicum, eucalyptus oil, and phellodendron bark, and preferably contains one or more selected from the group consisting of glycol salicylate, loxoprofen sodium, felbinac, l-menthol, and nonylic acid vanillylamide.

<11> The composition for external application according to any one of <1> to <10>, in which the SP value of the component (A) is preferably 15.00 or more, more preferably 18.00 or more, still more preferably 19.00 or more, and even more preferably 19.50 or more, and is preferably 35.00 or less, more preferably 27.00 or less, still more preferably 24.00 or less, and even more preferably 22.00 or less.

<12> The composition for external application according to any one of <1> to <11>, in which the component (B) is a film-forming water-insoluble polymer, and is preferably one or more water-insoluble polymers selected from the group consisting of an acrylic polymer, a cellulosic polymer, and a vinyl polymer.

<13> The composition for external application according to <12>, in which the acrylic polymer contains at least a structural unit derived from a monomer having a (meth)acrylic group, and is one or more selected from the group consisting of (acrylates/diacetoneacrylamide) copolymer, acrylamide-methoxypolyethylene glycol methacrylate copolymer, acrylic acid-octyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammoniumethyl methacrylate chloride copolymer, and methyl methacrylate-butyl methacrylate-dimethylaminoethyl methacrylate copolymer.

<14> The composition for external application according to <12>, in which the cellulosic polymer is one or more selected from the group consisting of methylcellulose, ethylcellulose, hypromellose, carboxymethylcellulose, hydroxyethylcellulose, hydroxypropylcellulose, hypromellose phthalate, and hydrophobized (C16-18) hydroxypropylmethylcellulose.

<15> The composition for external application according to <12>, in which the vinyl polymer is one or more selected from the group consisting of polyvinyl alcohol and polyvinyl butyral.

<16> The composition for external application according to any one of <1> to <15>, in which the component (B) is one or more selected from the group consisting of (acrylates/diacetoneacrylamide) copolymer, acrylamide-methoxypolyethylene glycol methacrylate copolymer, acrylic acid-octyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammoniumethyl methacrylate chloride copolymer, methyl methacrylate-butyl methacrylate-dimethylaminoethyl methacrylate copolymer, methylcellulose, ethylcellulose, hypromellose, carboxymethylcellulose, hydroxyethylcellulose, hydroxypropylcellulose, hypromellose phthalate, hydrophobized (C16-18) hydroxypropylmethylcellulose, polyvinyl alcohol, and polyvinyl butyral, and preferably one or more selected from the group consisting of (acrylates/diacetoneacrylamide) copolymer, acrylamide-methoxypolyethylene glycol methacrylate copolymer, ethylcellulose, hypromellose phthalate, and polyvinyl butyral.

<17> The composition for external application according to any one of <1> to <16>, in which the component (C) is one or more selected from the group consisting of a lipophilic base, a hydrophilic base, and an amphiphilic base, and preferably one or more selected from the group consisting of a lipophilic base and a hydrophilic base.

<18> The composition for external application according to any one of <1> to <17>, in which the content of the non-volatile base containing an unsaturated hydrocarbon chain having 16 or more carbon atoms in the component (C) is preferably 30% by mass or less, more preferably 10% by mass or less, still more preferably 5% by mass or less, even more preferably 1% by mass or less, and even more preferably 0% by mass.

<19> The composition for external application according to any one of <1> to <18>, in which the component (C) is one or more selected from the group consisting of an ester oil, a nonpolar oil, a polyol, and an alkanolamine.

<20> The composition for external application according to <19>, in which the ester oil is one or more selected from the group consisting of (i) a fatty acid monoester composed of a fatty acid and a monohydric alcohol, (ii) a fatty acid diester composed of a fatty acid and a dihydric alcohol, (iii) a dicarboxylic acid diester composed of a dicarboxylic acid and a monohydric alcohol, (iv) a tricarboxylic acid triester composed of a tricarboxylic acid and a monohydric alcohol, and (v) a glycerol fatty acid triester, preferably one or more selected from the group consisting of (i) a fatty acid monoester composed of a fatty acid and a monohydric alcohol, and (iii) a dicarboxylic acid diester composed of a dicarboxylic acid and a monohydric alcohol, more preferably one or more selected from the group consisting of a fatty acid monoester composed of a fatty acid and a monohydric alcohol having 1 or more and 24 or less carbon atoms, and a dicarboxylic acid diester composed of a dicarboxylic acid having 4 or more and 18 or less carbon atoms and a monohydric alcohol having 1 or more and 24 or less carbon atoms, and still more preferably one or more selected from the group consisting of isopropyl myristate, dibutyl adipate, diethyl sebacate, and diisopropyl sebacate.

<21> The composition for external application according to <19>, in which the nonpolar oil is one or more selected from the group consisting of a hydrocarbon oil, a silicone oil, and a fluorine oil, and preferably one or more selected from the group consisting of liquid paraffin, squalane, dimethylpolysiloxane, dimethylcyclopolysiloxane, methylphenylpolysiloxane, methylhydrogenpolysiloxane, higher alcohol-modified organopolysiloxane, fluoropolyether, and perfluoroalkyl ether silicone.

<22> The composition for external application according to <19>, in which the polyol is one or more selected from the group consisting of alkylene glycols, polyalkylene glycols, and glycerols, and preferably one or more selected from the group consisting of ethylene glycol, propylene glycol, 1,3-propanediol, 1,3-butylene glycol (1,3-butanediol), 1,2-pentanediol, diethylene glycol, dipropylene glycol, polyethylene glycol, polypropylene glycol, polyoxyethylene-polyoxypropylene glycol, glycerol, diglycerol, and triglycerol.

<23> The composition for external application according to <19>, in which the alkanolamine is preferably an alkanolamine having 9 or less carbon atoms, and more preferably 2 or more and 9 or less carbon atoms, and more preferably one or more selected from the group consisting of triethanolamine, diethanolamine, monoethanol amine, triisopropanolamine, diisopropanolamine, monoisopropanolamine, and 2-amino-2-methylpropanol.

<24> The composition for external application according to any one of <1> to <23>, in which the component (C) is one or more selected from the group consisting of isopropyl myristate, dibutyl adipate, diethyl sebacate, diisopropyl sebacate, propylene glycol, 1,3-butylene glycol, 1,2-pentanediol, dipropylene glycol, polyethylene glycol, polypropylene glycol, polyoxyethylene-polyoxypropylene glycol, glycerol, diglycerol, triethanolamine, monoethanolamine, diisopropanolamine, and 2-amino-2-methylpropanol, and preferably one or more selected from the group consisting of isopropyl myristate, diethyl sebacate, triethanolamine, diisopropanolamine, and 2-amino-2-methylpropanol.

<25> The composition for external application according to any one of <1> to <24>, in which the component (D) is one or more selected from the group consisting of an alcohol, a ketone, an ester, a hydrocarbon, and a silicone, preferably a lower alcohol, more preferably one or more selected from the group consisting of methanol, ethanol, n-propyl alcohol, isopropyl alcohol, n-butyl alcohol, isobutyl alcohol, sec-butyl alcohol, and tert-butyl alcohol, and still more preferably one or more selected from the group consisting of ethanol and isopropyl alcohol.

<26> The composition for external application according to any one of <1> to <25>, in which the content of the component (A) in the composition for external application is preferably 0.001% by mass or more, more preferably 0.005% by mass or more, still more preferably 0.01% by mass or more, even more preferably 0.1% by mass or more, and even more preferably 1.0% by mass or more, and is preferably 30% by mass or less, more preferably 20% by mass or less, still more preferably 15% by mass or less, even more preferably 10% by mass or less, and even more preferably 5.0% by mass or less.

<27> The composition for external application according to any one of <1> to <3> and <5> to <26>, in which the content of the component (B) in the composition for external application is preferably 0.01% by mass or more, more preferably 0.05% by mass or more, still more preferably 0.1% by mass or more, still more preferably 0.5% by mass or more, and still more preferably 1.0% by mass or more, and is preferably 30% by mass or less, more preferably 25% by mass or less, still more preferably 20% by mass or less, even more preferably 12% by mass or less, and even more preferably 5.0% by mass or less.

<28> The composition for external application according to any one of <1> to <4> and <6> to <27>, in which the content of the component (C) in the composition for external application is preferably 0.001% by mass or more, more preferably 0.005% by mass or more, still more preferably 0.01% by mass or more, still more preferably 0.1% by mass or more, and still more preferably 1.0% by mass or more, and is preferably 30% by mass or less, more preferably 20% by mass or less, still more preferably 15% by mass or less, even more preferably 10% by mass or less, even more preferably 8.0% by mass or less, and even more preferably 5.0% by mass or less.

<29> The composition for external application according to any one of <1> to <28>, in which the content of the component (D) in the composition for external application is preferably 10% by mass or more, more preferably 25% by mass or more, still more preferably 45% by mass or more, and even more preferably 65% by mass or more, and is preferably 95% by mass or less, more preferably 90% by mass or less, still more preferably 80% by mass or less, and even more preferably 75% by mass or less.

<30> The composition for external application according to any one of <1> and <4> to <29>, in which the content of the component (E) in the composition for external application is preferably 0.1% by mass or more, more preferably 0.5% by mass or more, still more preferably 5% by mass or more, and even more preferably 13% by mass or more, and is preferably 70% by mass or less, more preferably 50% by mass or less, still more preferably 30% by mass or less, even more preferably 25% by mass or less, even more preferably 22% by mass or less, even more preferably 21% by mass or less, and even more preferably 20% by mass or less.

<31> The composition for external application according to any one of <1> to <30>, in which the mass ratio [(A)/(B)] of the component (A) to the component (B) in the composition for external application is preferably 0.01 or more, more preferably 0.02 or more, still more preferably 0.03 or more, even more preferably 0.05 or more, even more preferably 0.10 or more, and even more preferably 0.15 or more, and is preferably 50 or less, more preferably 30 or less, still more preferably 20 or less, even more preferably 10 or less, even more preferably 5.0 or less, and even more preferably 3.0 or less.

<32> The composition for external application according to any one of <1> to <31>, in which the mass ratio [(A)/(C)] of the component (A) to the component (C) in the composition for external application is preferably 0.01 or more, more preferably 0.02 or more, still more preferably 0.03 or more, even more preferably 0.05 or more, even more preferably 0.10 or more, and even more preferably 0.15 or more, and is preferably 10 or less, more preferably 5.0 or less, still more preferably 3.0 or less, even more preferably 2.5 or less, even more preferably 2.0 or less, and even more preferably 1.5 or less.

<33> The composition for external application according to any one of <1> to <3> and <5> to <32>, in which the mass ratio [(C)/(B)] of the component (C) to the component (B) in the composition for external application is preferably 0.05 or more, more preferably 0.10 or more, still more preferably 0.20 or more, and even more preferably 0.50 or more, and is preferably 50 or less, more preferably 35 or less, still more preferably 20 or less, even more preferably 10 or less, even more preferably 5.0 or less, and even more preferably 3.5 or less.

<34> The composition for external application according to any one of <1> to <33>, in which the mass ratio [(E)/(D)] of the component (E) to the component (D) in the composition for external application is preferably 0.005 or more, more preferably 0.05 or more, still more preferably 0.10 or more, even more preferably 0.16 or more, and even more preferably 0.20 or more, and is preferably 3.0 or less, more preferably 2.0 or less, still more preferably 1.0 or less, even more preferably 0.80 or less, and even more preferably 0.40 or less.

<35> The composition for external application according to any one of <1> to <34>, in which the mass ratio [(B)/{(D) + (E)}] of the component (B) to the total content of the component (D) and the component (E) in the composition for external application is preferably 0.001 or more, more preferably 0.01 or more, and still more preferably 0.02 or more, and is preferably 1.0 or less, more preferably 0.50 or less, still more preferably 0.20 or less, even more preferably 0.12 or less, and even more preferably 0.10 or less.

<36> The composition for external application according to any one of <1> and <4> to <35>, in which the mass ratio [(A)/{(B) + (C)}] of the component (A) to the total amount of the component (B) and the component (C) in the composition for external application is preferably 0.001 or more, more preferably 0.005 or more, still more preferably 0.01 or more, even more preferably 0.05 or more, and even more preferably 0.10 or more, and is preferably 5.00 or less, more preferably 3.00 or less, still more preferably 2.50 or less, even more preferably 2.40 or less, even more preferably 2.00 or less, even more preferably 1.60 or less, even more preferably 1.00 or less, and even more preferably 0.60 or less.

<37> A lotion formulation, a gel formulation, an ointment formulation, a cream formulation, or a foam formulation including the composition for external application according to any one of <1> to <36>.

<38> An aerosol formulation or a pump spray-type formulation using the composition for external application according to any one of <1> to <36>.

<39> The aerosol formulation according to <38>, in which the aerosol formulation contains the composition for external application and a propellant, and the mass ratio of the composition for external application to the propellant is preferably in the range of 1:0.01 to 1:10, and more preferably in the range of 1:0.05 to 1:7.5.

<40> A method for using a composition for external application, including a step of applying to the skin a composition for external application containing (A) a medicinal ingredient, (B) a water-insoluble polymer, (C) a non-volatile base, (D) a volatile solvent, and (E) water, in which the composition has a viscosity at 25°C of 1.0 mPa -s or more, preferably 3.0 mPa -s or more, more preferably 5.0 mPa ·s or more, and still more preferably 10.0 mPa s or more, and 10,000 mPa -s or less, preferably 7,500 mPa s or less, more preferably 5,000 mPa -s or less, still more preferably 3,000 mPa ·s or less, even more preferably 2,000 mPa -s or less, even more preferably 1,000 mPa s or less, and even more preferably 700 mPa ·s or less, and then drying the composition.

<41> A method for using a composition for external application, including a step of applying to the skin a composition for external application containing (A) a medicinal ingredient, (B) a water-insoluble polymer, (C) a non-volatile base, (D) a volatile solvent, and (E) water, in which the content of the component (E) in the composition for external application is 0.1% by mass or more and 30% by mass or less, the mass ratio [(A)/{(B) + (C)}] of the component (A) to the total amount of the component (B) and the component (C) is 0.001 or more and 2.40 or less, and the viscosity at 25°C is 1.0 mPa s or more and 10,000 mPa s or less, and then drying the composition.

<42> A method for using a composition for external application, including a step of applying to the skin a composition for external application containing (A) a medicinal ingredient, (B) a water-insoluble polymer, (C) a non-volatile base, (D) a volatile solvent, and (E) water, in which the content of the component (E) in the composition for external application is more than 30% by mass and 70% by mass or less, the mass ratio [(A)/{(B) + (C)}] of the component (A) to the total amount of the component (B) and the component (C) is 0.001 or more and 2.40 or less, and the viscosity at 25°C is 1.0 mPa s or more and 10,000 mPa s or less, and then drying the composition.

<43> A method for using a composition for external application, including a step of applying to the skin a composition for external application containing (A) an anti-inflammatory analgesic component, (B) a water-insoluble polymer, (C) a non-volatile base, (D) a volatile solvent, and (E) water, in which the content of the component (B) in the composition for external application is 0.1% by mass or more and 15% by mass or less, the mass ratio [(C)/(B)] of the component (C) to the component (B) in the composition for external application is 0.05 or more and 3.5 or less, and the viscosity at 25°C is 1.0 mPa s or more and 10,000 mPa -s or less, and then drying the composition.

<44> A method for using a composition for external application, including a step of applying to the skin a composition for external application containing (A) one or more medicinal ingredients selected from the group consisting of glycol salicylate, diclofenac sodium, loxoprofen sodium, felbinac, indomethacin, piroxicam, flurbiprofen, methyl salicylate, ketoprofen, and nonylic acid vanillylamide, (B) a water-insoluble polymer, (C) a non-volatile base, (D) a volatile solvent, and (E) water, in which the content of the component (C) in the composition for external application is 5.0% by mass or less, and the viscosity at 25°C is 1.0 mPa s or more and 10,000 mPa s or less, and then drying the composition.

<45> The method for using a composition for external application according to any one of <40> to <44>, in which the composition for external application is applied to the skin by spraying.

<46> The method for using a composition for external application according to <45>, in which the method for spraying the composition for external application is a method using an aerosol, a pump spray, or an electrostatic spray device.

<47> The method for using a composition for external application according to <45> or <46>, in which the composition for external application is applied to the skin by spraying using an electrostatic spray device.

<48> A method for producing a coating film, including a step of applying the composition for external application according to any one of <1> to <36> to the skin by direct application or spraying.

<49> The method for producing a coating film according to <48>, in which the method for spraying the composition for external application is a method using an aerosol, a pump spray, or an electrostatic spray device.

<50> The method for producing a coating film according to <48> or <49>, in which the composition for external application is sprayed and applied to the skin by an electrostatic spray method using an electrostatic spray device.

Examples

Hereinafter, the present invention will be described by Examples, but the present invention is not limited to the scope of the Examples. In the present Examples, various measurements and evaluations were performed by the following methods.

(SP Value)

The SP value of the components used in the composition for external application of each Example was calculated based on the Hansen solubility parameter by using a solubility parameter calculation software (Hansen Solubility Parameters in Practice 4th Edition 4.1.03). In the calculation of the SP value, the specific gravity of the component (B) and the component (B') which were polymer components was calculated as 1.0.

(Viscosity)

The viscosity at 25°C of the composition for external application of each Example was measured using a vibration-type viscometer ("VM-10AL" manufactured by SEKONIC CORPORATION). In addition, the viscosity of a composition having a viscosity of more than 1000 mPa ·s was measured using a B-type rotational viscometer ("TV-10M" manufactured by Toki Sangyo Co., Ltd., rotor: TM3, rotation speed: 6 rpm).

(pH)

The pH at 25°C of the composition for external application of each Example was measured without dilution using a pH meter ("F-51" manufactured by HORIBA, Ltd.).

(Feeling of Use)

Regarding the feeling of use when 6 $\mu$L/cm$^2$ of the composition for external application of each Example was applied to the inside portion of the front arm using the tip of a pipet, 10 specialized panelists evaluated the following 6 items in 5 grades. The average value (rounded to the second decimal place) of the scores of the 10 panelists is shown in the tables. A higher score means a better evaluation result.

(1) Coating property to the skin

5: Extremely easy to spread, 4: Easy to spread, 3: Normal, 2: Slightly difficult to spread, 1: Very difficult to spread.

(2) Quick-drying property on application to the skin

5: Dry within 30 seconds, 4: Dry within 1 minute, 3: Dry within 2 minutes, 2: Dry within 10 minutes, 1: Not dry beyond 10 minutes.

(3) Appearance of the coating film

5: Transparent, 4: Slightly whitening observed but close to transparent, 3: Somewhat whitening observed but transparent, 2: Translucent, 1: Completely whitening.

(4) Sticky feeling of the coating film

5: Not sticky at all, 4: Stickiness is slightly felt, 3: Stickiness is somewhat felt, 2: Stickiness is significantly felt, 1: Very sticky.

(5) Tense feeling

5: No tense feeling at all, 4: Tense feeling is slightly felt, 3: Tense feeling is somewhat felt, 2: Tense feeling is significant, 1: Very tense feeling.

(6) Durability of the coating film

After the composition for external application was applied to the inside portion of the front arm, the specialized panelists performed daily living activities, and the formed coating film was visually observed after a certain period of time to confirm whether or not the coating film remained. The longest time during which remaining of the coating film was observed was scored according to the following criteria.

5: After 8 hours, 4: After 4 hours, 3: After 1 hour, 2: After 30 minutes, 1: After 10 minutes.

(Storage Stability)

10 g of the composition for external application of each Example was placed in a screw tube No. 4 (manufactured by Maruemu Corporation) and covered, and changes over time in appearance, odor, viscosity, and pH after storage for 1 week in constant temperature chambers of 0°C and 40°C were observed, and determination was performed according to the following criteria.

 A: There is no change in any of appearance, odor, viscosity, and pH.
 B: There is a slight change in any one or more of appearance, odor, viscosity, and pH.
 C: There is a change in any one of appearance, odor, viscosity, and pH.
 D: There is a change in two or more of appearance, odor, viscosity, and pH.

(Film-Forming Property)

The composition for external application of each Example was applied in an amount of 6 $\mu$L/cm$^2$ onto the surface of artificial leather ("SUPPLALE" manufactured by Ideatex Japan Co., Ltd.), and dried for 1 hour on a hotplate ("Ceramic Hotplate" manufactured by AS ONE Corporation) set at 32°C. The coated portion of the composition for external application was visually observed, and the film-forming property was evaluated according to the following criteria. A score of 3 or higher was accepted.

5: Uniform and sufficiently thick coating film was formed, 4: Uniform and thick coating film was formed, 3: Thin but uniform coating film was formed, 2: Coating film was present but not uniform, 1: Presence of a coating film could not be confirmed.

(Skin Permeation Amount of Medicinal Ingredient)

The composition for external application of each Example was applied to the surface (one side) of the horny layer of the pig ear set in a Franz diffusion cell (manufactured by PermeGear, Inc.) at 6 $\mu$L/cm$^2$, and the cumulative skin permeation amount ($\mu$g/cm$^2$) of the medicinal ingredient after 8 hours was measured. A larger value means a higher permeability of the medicinal ingredient into the skin.

(Crystallinity of Medicinal Ingredient)

The composition for external application of each Example was applied onto a slide glass at 5 $\mu$L/cm$^2$, and dried for 1 hour on a hotplate ("Ceramic Hotplate" manufactured by AS ONE Corporation) set at 32°C. The presence or absence of crystals in the formed coating film was evaluated by observation with a polarizing microscope, and judged according to the following criteria. A higher score means that the crystalline medicinal ingredient is less likely to precipitate and the release of the medicinal ingredient into the skin is good.

5: Crystals were not observed, 4: Crystals of less than about 10 pm were observed, 3: Crystals of about 10 pm or more and less than 50 pm were observed, 2: Crystals of about 50 pm or more and less than 100 pm were observed, 1: Crystals of about 100 pm or more were observed.

(Spinnability of Coating Film)

The composition for external application of each Example was applied onto a slide glass at 5 $\mu$L/cm$^2$, and dried for 1 hour on a hotplate ("Ceramic Hotplate" manufactured by AS ONE Corporation) set at 32°C. The spinnability was evaluated by touching the surface of the formed coating film with a finger, and the evaluation was performed according to the following criteria. A higher score means that the affinity of the medicinal ingredient with the coating film is not excessively high, so that the spinnability is low and the release of the medicinal ingredient into the skin is good.

5: No spinnability at all, 4: Almost no spinnability, 3: Slight spinnability, 2: Significant spinnability, 1: Extremely high spinnability.

(Appearance change during storage at a low temperature of -5°C)

10 g of each of the compositions shown in Table 7 was weighed into a clear vial and stored at -5°C for 6 hours. After storage, the vial was allowed to stand at 20°C for 20 minutes, and then the appearance of the composition in the vial was observed according to the following criteria. The background color of the vial during observation was black.

Clear: The composition is transparent, and the black color of the background color can be visually recognized without clouding.

Slightly Cloudy: The composition was slightly hazy, and the black color of the background color was slightly blurred.

Cloudy: The composition was hazy, and the black color of the background color was blurred.

Examples 1 to 42 and Comparative Examples 1 to 10 (Preparation and Evaluation of Compositions for External Application)

The component (B) or the component (B') and other components described in the tables were mixed with the component (C), the component (D), and the component (E) described in the tables and uniformly dissolved at 30°C, and then the component (A) was mixed and dissolved to prepare a composition for external application.

Using the obtained composition for external application, evaluations were performed by the above methods. The results are shown in Tables 1 to 7.

Note that the blending amount described in each table is an active component amount (parts by mass) of each component when "% by mass" is not described, and is an active component amount (% by mass) of each component when "% by mass" is described. In addition, in the tables, a water-soluble polymer which does not correspond to the component (B) is indicated as "component (B')".

Table 1

| | | (parts by mass) | SP value | Example 1 | 2 | 3 | 4 | 5 | 6 | 7 | 8 | 9 | 10 | 11 |
|---|---|---|---|---|---|---|---|---|---|---|---|---|---|---|
| (A) | *1 | Felbinac | 21.77 | 3.00 | | | | 3.00 | 3.00 | 3.00 | 3.00 | 3.00 | 3.00 | 3.00 |
| | *2 | Glycol salicylate | 26.55 | | 3.00 | | | | | | | | | |
| | *3 | Loxoprofen sodium | 20.18 | | | 3.00 | | | | | | | | |
| | *4 | 1-Menthol | 18.50 | | | | 3.00 | | | | | | | |
| | *5 | Nonylic acid vanillylamide | 25.97 | | | | | | | | | | | |
| (B) | *6 | (Acrylates/diacetoneacrylamide) copolymer | 21.81 | 3.00 | 3.00 | 3.00 | 3.00 | | | | | 3.00 | 3.00 | 3.00 |
| | *7 | Hypromellose phthalate | 23.99 | | | | | 3.00 | | | | | | |
| | *8 | Acrylamide-methoxypolyethylene glycol methacrylate copolymer | 22.33 | | | | | | 3.00 | | | | | |
| | *9 | Ethylcellulose | 22.96 | | | | | | | 3.00 | | | | |
| | *10 | Polyvinyl butyral | 20.90 | | | | | | | | 3.00 | | | |
| (B') | *11 | Methyl vinyl ether-maleic anhydride | 28.85 | | | | | | | | | | | |
| | *12 | Isopropyl myristate | 16.29 | 1.00 | 1.00 | 1.00 | 1.00 | 1.00 | 1.00 | 1.00 | 1.00 | | 1.00 | 1.00 |
| | *13 | Diethyl sebacate | 17.44 | | | | | | | | | 1.00 | | |
| | *14 | Oleyl alcohol | 18.19 | | | | | | | | | | | |
| | *15 | Squalane | 15.20 | | | | | | | | | | | |
| (C) | *16 | Diisopropanolamine | 18.46 | 2.00 | 2.00 | 2.00 | 2.00 | 2.00 | 2.00 | 2.00 | 2.00 | 2.00 | | 2.00 |
| | *17 | Triethanolamine | 28.78 | | | | | | | | | | 2.00 | |
| | *18 | 2-Amino-2-methylpropanol | 22.54 | 0.40 | 0.40 | 0.40 | 0.40 | 0.40 | 0.40 | 0.40 | 0.40 | 0.40 | 0.40 | 0.40 |

(continued)

| (parts by mass) | | | SP value | Example | | | | | | | | | | |
|---|---|---|---|---|---|---|---|---|---|---|---|---|---|---|
| | | | | 1 | 2 | 3 | 4 | 5 | 6 | 7 | 8 | 9 | 10 | 11 |
| (D) | | Ethanol | | 65.00 | 65.00 | 65.00 | 65.00 | 65.00 | 65.00 | 65.00 | 65.00 | 65.00 | 65.00 | |
| | | Isopropyl alcohol | | | | | | | | | | | | 65.00 |
| (E) | | Purified water | | balance | balance | balance | balance | balance | balance | balance | balance | balance | balance | balance |
| Other | *19 | Xylitol | 36.28 | | | | | | | | | | | |
| Total amount | | | | 90.00 | 90.00 | 90.00 | 90.00 | 90.00 | 90.00 | 90.00 | 90.00 | 90.00 | 90.00 | 90.00 |
| | | | | Example | | | | | | | | | | |
| | | | | 1 | 2 | 3 | 4 | 5 | 6 | 7 | 8 | 9 | 10 | 11 |
| Component (A) content (% by mass) | | | | 3.33 | 3.33 | 3.33 | 3.33 | 3.33 | 3.33 | 3.33 | 3.33 | 3.33 | 3.33 | 3.33 |
| Component (B) content (% by mass) | | | | 3.33 | 3.33 | 3.33 | 3.33 | 3.33 | 3.33 | 3.33 | 3.33 | 3.33 | 3.33 | 3.33 |
| Component (C) content (% by mass) | | | | 3.78 | 3.78 | 3.78 | 3.78 | 3.33 | 3.33 | 3.33 | 3.33 | 3.78 | 3.78 | 3.78 |
| Component (D) content (% by mass) | | | | 72.22 | 72.22 | 72.22 | 72.22 | 72.22 | 72.22 | 72.22 | 72.22 | 72.22 | 72.22 | 72.22 |
| Component (E) content (% by mass) | | | | 17.33 | 17.33 | 17.33 | 17.33 | 17.78 | 17.78 | 17.78 | 17.78 | 17.33 | 17.33 | 17.33 |
| Components (A) to (E) content (% by mass) | | | | 100.00 | 100.00 | 100.00 | 100.00 | 100.00 | 100.00 | 100.00 | 100.00 | 100.00 | 100.00 | 100.00 |
| (A)/(B) | | | | 1.00 | 1.00 | 1.00 | 1.00 | 1.00 | 1.00 | 1.00 | 1.00 | 1.00 | 1.00 | 1.00 |
| (A)/(C) | | | | 0.88 | 0.88 | 0.88 | 0.88 | 1.00 | 1.00 | 1.00 | 1.00 | 0.88 | 0.88 | 0.88 |
| (C)/(B) | | | | 1.13 | 1.13 | 1.13 | 1.13 | 1.00 | 1.00 | 1.00 | 1.00 | 1.13 | 1.13 | 1.13 |
| (E)/(D) | | | | 0.24 | 0.24 | 0.24 | 0.24 | 0.25 | 0.25 | 0.25 | 0.25 | 0.24 | 0.24 | 0.24 |
| (B)/{(D)+(E)} | | | | 0.037 | 0.037 | 0.037 | 0.037 | 0.037 | 0.037 | 0.037 | 0.037 | 0.037 | 0.037 | 0.037 |
| (A)/{(B)+(C)} | | | | 0.47 | 0.47 | 0.47 | 0.47 | 0.50 | 0.50 | 0.50 | 0.50 | 0.47 | 0.47 | 0.47 |
| Viscosity (mPa·s) | | | | 51.5 | 46.5 | 53.2 | 55.6 | 42.1 | 72.9 | 73.9 | 64.1 | 55.9 | 52.4 | 53.6 |
| $SP_{B+C}$ | | | | 19.82 | 19.82 | 19.82 | 19.82 | 20.68 | 19.88 | 20.18 | 19.19 | 20.10 | 22.71 | 19.82 |
| $\Delta(SP_A-SP_{B+C})$ *20 | | | | 1.95 | 6.73 | 0.36 | 1.32 | 1.09 | 1.89 | 1.59 | 2.58 | 1.67 | 0.94 | 1.95 |

(continued)

| | | Example | | | | | | | | | | |
|---|---|---|---|---|---|---|---|---|---|---|---|---|
| | | 1 | 2 | 3 | 4 | 5 | 6 | 7 | 8 | 9 | 10 | 11 |
| Feeling of use | Coating property to skin | 5.0 | 4.8 | 4.5 | 4.7 | 4.6 | 4.5 | 4.8 | 5.0 | 4.8 | 4.9 | 4.5 |
| | Quick-drying property on application to skin | 4.8 | 4.7 | 4.5 | 5.0 | 4.8 | 4.5 | 4.9 | 5.0 | 4.7 | 4.8 | 4.6 |
| | Appearance of coating film | 5.0 | 4.8 | 5.0 | 4.9 | 4.6 | 4.9 | 4.6 | 4.6 | 4.7 | 5.0 | 4.9 |
| | Sticky feeling of coating film | 5.0 | 5.0 | 4.8 | 4.9 | 5.0 | 4.7 | 4.8 | 4.6 | 5.0 | 4.5 | 4.8 |
| | Tense feeling | 4.9 | 4.7 | 4.8 | 4.6 | 5.0 | 4.7 | 4.7 | 4.8 | 4.8 | 4.5 | 4.9 |
| | Durability of coating film | 4.7 | 4.5 | 5.0 | 5.0 | 4.5 | 5.0 | 4.9 | 4.8 | 4.5 | 5.0 | 4.8 |
| Storage stability (0°C, one week) | | A | A | A | A | A | A | A | A | A | A | A |
| Storage stability (40°C, one week) | | A | A | A | A | A | A | A | A | A | A | A |
| Film-forming property | | 5 | 5 | 5 | 5 | 5 | 5 | 5 | 5 | 5 | 5 | 5 |
| Skin permeation amount of medicinal ingredient ($\mu g/cm^2$) | | 7.43 | 68.9 | 1.02 | 2.54 | 6.17 | 6.95 | 6.51 | 7.29 | 5.04 | 5.50 | 6.19 |
| Crystallinity evaluation of medicinal ingredient | | 5 | 5 | 5 | 5 | 5 | 5 | 5 | 5 | 5 | 5 | 5 |
| Spinnability of coating film | | 5 | 5 | 5 | 5 | 5 | 5 | 5 | 5 | 5 | 5 | 5 |

Table 2

| (parts by mass) | | | SP value | Example | | | | | | | | | |
|---|---|---|---|---|---|---|---|---|---|---|---|---|---|
| | | | | 12 | 13 | 14 | 15 | 16 | 17 | 18 | 19 | 20 | 21 |
| (A) | *1 | Felbinac | 21.77 | 0.50 | 5.00 | 10.00 | 15.00 | | | | | | |
| | *2 | Glycol salicylate | 26.55 | | | | | 1.50 | 10.00 | | | | |
| | *3 | Loxoprofen sodium | 20.18 | | | | | | | 0.10 | 5.00 | | |
| | *4 | l-Menthol | 18.50 | | | | | | | | | 0.50 | 10.00 |
| | *5 | Nonylic acid vanillylamide | 25.97 | | | | | | | | | | |
| (B) | *6 | (Acrylates/ diacetoneacrylamide) copolymer | 21.81 | 3.00 | 3.00 | 3.00 | 3.00 | 3.00 | 3.00 | 3.00 | 3.00 | 3.00 | 3.00 |
| | *7 | Hypromellose phthalate | 23.99 | | | | | | | | | | |
| | *8 | Acrylamide-methoxypolyethylene glycol methacrylate copolymer | 22.33 | | | | | | | | | | |
| | *9 | Ethylcellulose | 22.96 | | | | | | | | | | |
| | *10 | Polyvinyl butyral | 20.90 | | | | | | | | | | |
| (B') | *11 | Methyl vinyl ether-maleic anhydride | 28.85 | | | | | | | | | | |
| (C) | *12 | Isopropyl myristate | 16.29 | 1.00 | 1.00 | 1.00 | 1.00 | 1.00 | 1.00 | 1.00 | 1.00 | 1.00 | 1.00 |
| | *13 | Diethyl sebacate | 17.44 | | | | | | | | | | |
| | *14 | Oleyl alcohol | 18.19 | | | | | | | | | | |
| | *15 | Squalane | 15.20 | | | | | | | | | | |
| | *16 | Diisopropanolamine | 18.46 | 2.00 | 2.00 | 2.00 | 2.00 | 2.00 | 2.00 | 2.00 | 2.00 | 2.00 | 2.00 |
| | *17 | Triethanolamine | 28.78 | | | | | | | | | | |
| | *18 | 2-Amino-2-methylpropanol | 22.54 | 0.40 | 0.40 | 0.40 | 0.40 | 0.40 | 0.40 | 0.40 | 0.40 | 0.40 | 0.40 |

EP 4 285 931 A1

26

(continued)

| (parts by mass) | | | SP value | Example | | | | | | | | | |
|---|---|---|---|---|---|---|---|---|---|---|---|---|---|
| | | | | 12 | 13 | 14 | 15 | 16 | 17 | 18 | 19 | 20 | 21 |
| (D) | | Ethanol | | 65.00 | 65.00 | 65.00 | 65.00 | 65.00 | 65.00 | 65.00 | 65.00 | 65.00 | 65.00 |
| | | Isopropyl alcohol | | | | | | | | | | | |
| (E) | | Purified water | | balance | balance | balance | balance | balance | balance | balance | balance | balance | balance |
| Other | *19 | Xylitol | 36.28 | | | | | | | | | | |
| Total amount | | | | 90.00 | 90.00 | 90.00 | 90.00 | 90.00 | 90.00 | 90.00 | 90.00 | 90.00 | 90.00 |

| | Example | | | | | | | | | |
|---|---|---|---|---|---|---|---|---|---|---|
| | 12 | 13 | 14 | 15 | 16 | 17 | 18 | 19 | 20 | 21 |
| Component (A) content (% by mass) | 0.56 | 5.56 | 11.11 | 16.67 | 1.67 | 11.11 | 0.11 | 5.56 | 0.56 | 11.11 |
| Component (B) content (% by mass) | 3.33 | 3.33 | 3.33 | 3.33 | 3.33 | 3.33 | 3.33 | 3.33 | 3.33 | 3.33 |
| Component (C) content (% by mass) | 3.78 | 3.78 | 3.78 | 3.78 | 3.78 | 3.78 | 3.78 | 3.78 | 3.78 | 3.78 |
| Component (D) content (% by mass) | 72.22 | 72.22 | 72.22 | 72.22 | 72.22 | 72.22 | 72.22 | 72.22 | 72.22 | 72.22 |
| Component (E) content (% by mass) | 20.11 | 15.11 | 9.56 | 4.00 | 19.00 | 9.56 | 20.56 | 15.11 | 20.11 | 9.56 |
| Components (A) to (E) content (% by mass) | 100.00 | 100.00 | 100.00 | 100.00 | 100.00 | 100.00 | 100.00 | 100.00 | 100.00 | 100.00 |
| (A)/(B) | 0.17 | 1.67 | 3.33 | 5.00 | 0.50 | 3.33 | 0.03 | 1.67 | 0.17 | 3.33 |
| (A)/(C) | 0.15 | 1.47 | 2.94 | 4.41 | 0.44 | 2.94 | 0.03 | 1.47 | 0.15 | 2.94 |
| (C)/(B) | 1.13 | 1.13 | 1.13 | 1.13 | 1.13 | 1.13 | 1.13 | 1.13 | 1.13 | 1.13 |
| (E)/(D) | 0.28 | 0.21 | 0.13 | 0.06 | 0.26 | 0.13 | 0.28 | 0.21 | 0.28 | 0.13 |
| (B)/{(D)+(E)} | 0.036 | 0.038 | 0.041 | 0.044 | 0.037 | 0.041 | 0.036 | 0.038 | 0.036 | 0.041 |
| (A)/{(B)+(C)} | 0.08 | 0.78 | 1.56 | 2.34 | 0.23 | 1.56 | 0.02 | 0.78 | 0.08 | 1.56 |
| Viscosity (mPa·s) | 50.7 | 56.8 | 57.1 | 59.4 | 48.9 | 43.2 | 56.8 | 50.2 | 57.2 | 53.7 |
| $SP_{B+C}$ | 19.82 | 19.82 | 19.82 | 19.82 | 19.82 | 19.82 | 19.82 | 19.82 | 19.82 | 19.82 |
| $\Delta(SP_A - SP_{B+C})$ *20 | 1.95 | 1.95 | 1.95 | 1.95 | 6.73 | 6.73 | 0.36 | 0.36 | 1.32 | 1.32 |

(continued)

| | | Example | | | | | | | | | |
|---|---|---|---|---|---|---|---|---|---|---|---|
| | | 12 | 13 | 14 | 15 | 16 | 17 | 18 | 19 | 20 | 21 |
| Feeling of use | Coating property to skin | 4.6 | 4.7 | 4.6 | 4.8 | 4.8 | 4.2 | 4.5 | 4.6 | 4.8 | 4.6 |
| | Quick-drying property on application to skin | 4.8 | 4.8 | 4.6 | 4.4 | 4.9 | 4.7 | 4.5 | 4.8 | 4.8 | 4.8 |
| | Appearance of coating film | 4.5 | 4.8 | 4.7 | 4.9 | 4.7 | 4.8 | 4.2 | 5.0 | 4.7 | 5.0 |
| | Sticky feeling of coating film | 4.7 | 4.5 | 4.5 | 4.5 | 4.9 | 5.0 | 4.7 | 4.8 | 4.6 | 4.8 |
| | Tense feeling | 4.7 | 4.5 | 4.5 | 4.5 | 4.9 | 4.8 | 4.6 | 4.8 | 4.5 | 4.8 |
| | Durability of coating film | 4.9 | 5.0 | 4.3 | 3.8 | 4.5 | 4.3 | 4.9 | 4.9 | 4.9 | 4.9 |
| Storage stability (0°C, one week) | | A | B | B | B | A | B | A | B | A | A |
| Storage stability (40°C, one week) | | A | A | A | A | A | B | A | A | A | B |
| Film-forming property | | 5 | 5 | 5 | 5 | 5 | 5 | 5 | 5 | 5 | 5 |
| Skin permeation amount of medicinal ingredient ($\mu$g/cm$^2$) | | 3.06 | 10.4 | 12.7 | 15.9 | - | - | - | - | - | - |
| Crystallinity evaluation of medicinal ingredient | | 5 | 4 | 4 | 3 | 5 | 5 | 5 | 4 | 5 | 4 |
| Spinnability of coating film | | 5 | 5 | 5 | 5 | 5 | 5 | 5 | 5 | 5 | 5 |
| "-" indicates that evaluation is not performed. | | | | | | | | | | | |

Table 3

| (parts by mass) | | | SP value | Example | | | | | | | |
|---|---|---|---|---|---|---|---|---|---|---|---|
| | | | | 22 | 23 | 24 | 25 | 26 | 27 | 28 | 29 |
| (A) | *1 | Felbinac | 21.77 | 3.00 | 3.00 | 3.00 | 3.00 | 3.00 | 3.00 | 3.00 | 3.00 |
| | *2 | Glycol salicylate | 26.55 | | | | | | | | |
| | *3 | Loxoprofen sodium | 20.18 | | | | | | | | |
| | *4 | l-Menthol | 18.50 | | | | | | | | |
| | *5 | Nonylic acid vanillylamide | 25.97 | | | | | | | | |
| (B) | *6 | (Acrylates/diacetoneacrylamide) copolymer | 21.81 | 0.10 | 1.00 | 10.00 | 15.00 | | | | |
| | *7 | Hypromellose phthalate | 23.99 | | | | | 0.10 | 1.00 | 10.00 | 15.00 |
| | *8 | Acrylamide-methoxypolyethylene glycol methacrylate copolymer | 22.33 | | | | | | | | |
| | *9 | Ethylcellulose | 22.96 | | | | | | | | |
| | *10 | Polyvinyl butyral | 20.90 | | | | | | | | |
| (B') | *11 | Methyl vinyl ether-maleic anhydride | 28.85 | | | | | | | | |
| (C) | *12 | Isopropyl myristate | 16.29 | 1.00 | 1.00 | 1.00 | 1.00 | 1.00 | 1.00 | 1.00 | 1.00 |
| | *13 | Diethyl sebacate | 17.44 | | | | | | | | |
| | *14 | Oleyl alcohol | 18.19 | | | | | | | | |
| | *15 | Squalane | 15.20 | | | | | | | | |
| | *16 | Diisopropanolamine | 18.46 | 2.00 | 2.00 | 2.00 | 2.00 | 2.00 | 2.00 | 2.00 | 2.00 |
| | *17 | Triethanolamine | 28.78 | | | | | | | | |
| | *18 | 2-Amino-2-methylpropanol | 22.54 | 0.013 | 0.13 | 1.33 | 2.00 | | | | |
| (D) | | Ethanol | | 65.00 | 65.00 | 65.00 | 65.00 | 65.00 | 65.00 | 65.00 | 65.00 |
| | | Isopropyl alcohol | | | | | | | | | |
| (E) | | Purified water | | balance | balance | balance | balance | balance | balance | balance | balance |
| Other | *19 | Xylitol | 36.28 | | | | | | | | |
| Total amount | | | | 90.00 | 90.00 | 90.00 | 90.00 | 90.00 | 90.00 | 90.00 | 90.00 |

EP 4 285 931 A1

29

(continued)

| | Example | | | | | | | |
|---|---|---|---|---|---|---|---|---|
| | 22 | 23 | 24 | 25 | 26 | 27 | 28 | 29 |
| Component (A) content (% by mass) | 3.33 | 3.33 | 3.33 | 3.33 | 3.33 | 3.33 | 3.33 | 3.33 |
| Component (B) content (% by mass) | 0.11 | 1.11 | 11.11 | 16.67 | 0.11 | 1.11 | 11.11 | 16.67 |
| Component (C) content (% by mass) | 3.35 | 3.48 | 4.81 | 5.56 | 3.33 | 3.33 | 3.33 | 3.33 |
| Component (D) content (% by mass) | 72.22 | 72.22 | 72.22 | 72.22 | 72.22 | 72.22 | 72.22 | 72.22 |
| Component (E) content (% by mass) | 20.99 | 19.85 | 8.52 | 2.22 | 21.00 | 20.00 | 10.00 | 4.44 |
| Components (A) to (E) content (% by mass) | 100.00 | 100.00 | 100.00 | 100.00 | 100.00 | 100.00 | 100.00 | 100.00 |
| (A)/(B) | 30.00 | 3.00 | 0.30 | 0.20 | 30.00 | 3.00 | 0.30 | 0.20 |
| (A)/(C) | 1.00 | 0.96 | 0.69 | 0.60 | 1.00 | 1.00 | 1.00 | 1.00 |
| (C)/(B) | 30.13 | 3.13 | 0.43 | 0.33 | 30.00 | 3.00 | 0.30 | 0.20 |
| (E)/(D) | 0.29 | 0.27 | 0.12 | 0.03 | 0.29 | 0.28 | 0.14 | 0.06 |
| (B)/{(D)+(E)} | 0.001 | 0.012 | 0.138 | 0.224 | 0.001 | 0.012 | 0.135 | 0.217 |
| (A)/{(B)+(C)} | 0.96 | 0.73 | 0.21 | 0.15 | 0.97 | 0.75 | 0.23 | 0.17 |
| Viscosity (mPa·s) | 1.3 | 21.8 | 4250 | 6500 | 1.5 | 29.3 | 4500 | 9250 |
| $SP_{B+C}$ | 17.77 | 18.74 | 20.95 | 21.22 | 17.82 | 19.12 | 22.43 | 22.86 |
| $\Delta(SP_A - SP_{B+C})$ *20 | 4.00 | 3.03 | 0.82 | 0.55 | 3.95 | 2.65 | 0.66 | 1.09 |
| Feeling of use | Coating property to skin Quick-drying property on application to skin | 4.3 | 4.5 | 4.5 | 4.3 | 4.2 | 4.4 | 4.8 | 4.1 |
| | | 4.9 | 4.8 | 3.9 | 3.6 | 4.9 | 4.9 | 4.3 | 3.5 |
| | Appearance of coating film | 4.6 | 4.6 | 4.3 | 3.8 | 4.6 | 4.8 | 3.8 | 3.7 |
| | Sticky feeling of coating film | 4.5 | 4.7 | 4.9 | 4.9 | 4.5 | 4.8 | 4.8 | 4.8 |
| | Tense feeling | 4.8 | 4.8 | 5.0 | 5.0 | 5.0 | 4.8 | 4.6 | 4.6 |
| | Durability of coating film | 3.6 | 4.4 | 5.0 | 4.9 | 3.8 | 4.4 | 4.8 | 4.8 |
| Storage stability (0°C, one week) | A | A | B | B | A | A | B | C |
| Storage stability (40°C, one week) | A | A | A | A | A | A | A | A |
| Film-forming property | 4 | 4 | 5 | 5 | 4 | 4 | 5 | 5 |

EP 4 285 931 A1

(continued)

| | Example | | | | | | | |
|---|---|---|---|---|---|---|---|---|
| | 22 | 23 | 24 | 25 | 26 | 27 | 28 | 29 |
| Skin permeation amount of medicinal ingredient ($\mu$g/cm$^2$) | - | - | - | - | 3.13 | 8.19 | 4.02 | 5.46 |
| Crystallinity evaluation of medicinal ingredient | 4 | 5 | 5 | 5 | 3 | 5 | 5 | 5 |
| Spinnability of coating film | 5 | 5 | 4 | 4 | 5 | 5 | 4 | 5 |
| "-" indicates that evaluation is not performed. | | | | | | | | |

Table 4

| | (parts by mass) | SP value | Example | | | | | | | |
|---|---|---|---|---|---|---|---|---|---|---|
| | | | 30 | 31 | 32 | 33 | 34 | 35 | 36 | 37 |
| (A) | *1 Felbinac | 21.77 | 3.00 | 3.00 | 3.00 | 3.00 | 3.00 | 3.00 | 3.00 | 3.00 |
| | *2 Glycol salicylate | 26.55 | | | | | | | | |
| | *3 Loxoprofen sodium | 20.18 | | | | | | | | |
| | *4 l-Menthol | 18.50 | | | | | | | | |
| | *5 Nonylic acid vanillylamide | 25.97 | | | | | | | | |
| (B) | *6 (Acrylates/diacetoneacrylamide) copolymer | 21.81 | 3.00 | 3.00 | 3.00 | 3.00 | 3.00 | 3.00 | 3.00 | 3.00 |
| | *7 Hypromellose phthalate | 23.99 | | | | | | | | |
| | *8 Acrylamide-methoxypolyethylene glycol methacrylate copolymer | 22.33 | | | | | | | | |
| | *9 Ethylcellulose | 22.96 | | | | | | | | |
| | *10 Polyvinyl butyral | 20.90 | | | | | | | | |
| (B') | *11 Methyl vinyl ether-maleic anhydride | 28.85 | | | | | | | | |
| | *12 Isopropyl myristate | 16.29 | 0.10 | 0.50 | 5.00 | 10.00 | 1.00 | 1.00 | 1.00 | 1.00 |
| | *13 Diethyl sebacate | 17.44 | | | | | | | | |
| | *14 Oleyl alcohol | 18.19 | | | | | | | | |
| (C) | *15 Squalane | 15.20 | | | | | | | | |
| | *16 Diisopropanolamine | 18.46 | 2.00 | 2.00 | 2.00 | 2.00 | 2.00 | 2.00 | 2.00 | 2.00 |
| | *17 Triethanolamine | 28.78 | | | | | | | | |
| | *18 2-Amino-2-methylpropanol | 22.54 | 0.40 | 0.40 | 0.40 | 0.40 | 0.40 | 0.40 | 0.40 | 0.40 |
| (D) | Ethanol | | 65.00 | 65.00 | 65.00 | 65.00 | 30.00 | 50.00 | 70.00 | 80.00 |
| | Isopropyl alcohol | | | | | | | | | |
| (E) | Purified water | | balance | balance | balance | balance | balance | balance | balance | balance |
| Other | *19 Xylitol | 36.28 | | | | | | | | |
| Total amount | | | 90.00 | 90.00 | 90.00 | 90.00 | 90.00 | 90.00 | 90.00 | 90.00 |

(continued)

| | Example | | | | | | | |
|---|---|---|---|---|---|---|---|---|
| | 30 | 31 | 32 | 33 | 34 | 35 | 36 | 37 |
| Component (A) content (% by mass) | 3.33 | 3.33 | 3.33 | 3.33 | 3.33 | 3.33 | 3.33 | 3.33 |
| Component (B) content (% by mass) | 3.33 | 3.33 | 3.33 | 3.33 | 3.33 | 3.33 | 3.33 | 3.33 |
| Component (C) content (% by mass) | 2.78 | 3.22 | 8.22 | 13.78 | 3.78 | 3.78 | 3.78 | 3.78 |
| Component (D) content (% by mass) | 72.22 | 72.22 | 72.22 | 72.22 | 33.33 | 55.56 | 77.78 | 88.89 |
| Component (E) content (% by mass) | 18.33 | 17.89 | 12.89 | 7.33 | 56.22 | 34.00 | 11.78 | 0.67 |
| Components (A) to (E) content (% by mass) | 100.00 | 100.00 | 100.00 | 100.00 | 100.00 | 100.00 | 100.00 | 100.00 |
| (A)/(B) | 1.00 | 1.00 | 1.00 | 1.00 | 1.00 | 1.00 | 1.00 | 1.00 |
| (A)/(C) | 1.20 | 1.03 | 0.41 | 0.24 | 0.88 | 0.88 | 0.88 | 0.88 |
| (C)/(B) | 0.83 | 0.97 | 2.47 | 4.13 | 1.13 | 1.13 | 1.13 | 1.13 |
| (E)/(D) | 0.25 | 0.25 | 0.18 | 0.10 | 1.69 | 0.61 | 0.15 | 0.008 |
| (B)/{(D)+(E)} | 0.037 | 0.037 | 0.039 | 0.042 | 0.037 | 0.037 | 0.037 | 0.037 |
| (A)/{(B)+(C)} | 0.55 | 0.51 | 0.29 | 0.19 | 0.47 | 0.47 | 0.47 | 0.47 |
| Viscosity (mPa·s) | 45.7 | 41.9 | 46.8 | 40.2 | 60.1 | 46.8 | 28.9 | 15.4 |
| $SP_{B+C}$ | 20.53 | 20.18 | 18.31 | 17.61 | 19.82 | 19.82 | 19.82 | 19.82 |
| $\Delta(SP_A-SP_{B+C})$ *20 | 1.24 | 1.59 | 3.46 | 4.16 | 1.95 | 1.95 | 1.95 | 1.95 |
| Feeling of use | Coating property to skin | 4.8 | 4.7 | 4.9 | 4.6 | 3.8 | 4.4 | 4.4 | 4.1 |
| | Quick-drying property on application to skin | 4.6 | 4.8 | 4.8 | 4.7 | 4.1 | 4.3 | 4.7 | 5.0 |
| | Appearance of coating film | 4.9 | 4.7 | 4.5 | 4.8 | 4.6 | 4.7 | 4.8 | 5.0 |
| | Sticky feeling of coating film | 4.7 | 4.5 | 4.2 | 4.1 | 4.9 | 4.5 | 4.7 | 4.8 |
| | Tense feeling | 3.9 | 4.4 | 4.6 | 4.9 | 4.6 | 4.8 | 4.6 | 4.8 |
| | Durability of coating film | 4.8 | 4.6 | 4.3 | 4.2 | 5.0 | 4.9 | 4.8 | 4.9 |
| Storage stability (0°C, one week) | A | A | A | A | B | B | A | A |
| Storage stability (40°C, one week) | A | A | A | A | A | A | A | A |
| Film-forming property | 5 | 5 | 4 | 3 | 5 | 5 | 4 | 4 |

(continued)

| | Example | | | | | | | |
|---|---|---|---|---|---|---|---|---|
| | 30 | 31 | 32 | 33 | 34 | 35 | 36 | 37 |
| Skin permeation amount of medicinal ingredient ($\mu$g/cm$^2$) | - | - | - | - | - | - | - | - |
| Crystallinity evaluation of medicinal ingredient | 5 | 5 | 4 | 4 | 5 | 5 | 5 | 5 |
| Spinnability of coating film | 5 | 5 | 5 | 5 | 5 | 5 | 5 | 5 |
| "-" indicates that evaluation is not performed. | | | | | | | | |

Table 5

| (parts by mass) | | | | SP value | Example | | | | |
|---|---|---|---|---|---|---|---|---|---|
| | | | | | 38 | 39 | 40 | 41 | 42 |
| (A) | *1 | | Felbinac | 21.77 | 3.00 | 3.00 | 3.00 | 3.00 | 3.00 |
| | *2 | | Glycol salicylate | 26.55 | | | | | |
| | *3 | | Loxoprofen sodium | 20.18 | | | | | |
| | *4 | | l-Menthol | 18.50 | | 3.00 | | | |
| | *5 | | Nonylic acid vanillylamide | 25.97 | | 0.012 | | | |
| (B) | *6 | | (Acrylates/ diacetoneacrylamide) copolymer | 21.81 | 3.00 | 3.00 | 1.50 | 3.00 | 3.00 |
| | *7 | | Hypromellose phthalate | 23.99 | | | 1.50 | | |
| | *8 | | Acrylamide-methoxypolyethylene glycol methacrylate copolymer | 22.33 | | | | | |
| | *9 | | Ethylcellulose | 22.96 | | | | | |
| | *10 | | Polyvinyl butyral | 20.90 | | | | | |
| (B') | *11 | | Methyl vinyl ether-maleic anhydride | 28.85 | | | | | |
| (C) | *12 | | Isopropyl myristate | 16.29 | | 1.00 | 1.00 | 0.50 | 1.00 |
| | *13 | | Diethyl sebacate | 17.44 | | | | 0.50 | |
| | *14 | | Oleyl alcohol | 18.19 | 1.00 | | | | |
| | *15 | | Squalane | 15.20 | | | | | |
| | *16 | | Diisopropanolamine | 18.46 | 2.00 | 2.00 | 2.00 | 1.00 | 2.00 |
| | *17 | | Triethanolamine | 28.78 | | | | 1.00 | |
| | *18 | | 2-Amino-2-methylpropanol | 22.54 | 0.40 | 0.40 | 0.20 | 0.40 | 0.40 |
| (D) | | | Ethanol | | 65.00 | 65.00 | 65.00 | 65.00 | 35.00 |
| | | | Isopropyl alcohol | | | | | | 30.00 |
| (E) | | | Purified water | | balance | balance | balance | balance | balance |
| Other | *19 | | Xylitol | 36.28 | | | | | |
| Total amount | | | | | 90.00 | 90.00 | 90.00 | 90.00 | 90.00 |

| | Example | | | | |
|---|---|---|---|---|---|
| | 38 | 39 | 40 | 41 | 42 |
| Component (A) content (% by mass) | 3.33 | 6.68 | 3.33 | 3.33 | 3.33 |
| Component (B) content (% by mass) | 3.33 | 3.33 | 3.33 | 3.33 | 3.33 |
| Component (C) content (% by mass) | 3.78 | 3.78 | 3.56 | 3.78 | 3.78 |
| Component (D) content (% by mass) | 72.22 | 72.22 | 72.22 | 72.22 | 72.22 |
| Component (E) content (% by mass) | 17.33 | 13.99 | 17.56 | 17.33 | 17.33 |
| Components (A) to (E) content (% by mass) | 100.00 | 100.00 | 100.00 | 100.00 | 100.00 |
| (A)/(B) | 1.00 | 2.00 | 1.00 | 1.00 | 1.00 |
| (A)/(C) | 0.88 | 1.77 | 0.94 | 0.88 | 0.88 |

(continued)

| | | Example | | | | |
|---|---|---|---|---|---|---|
| | | 38 | 39 | 40 | 41 | 42 |
| (C)/(B) | | 1.13 | 1.13 | 1.07 | 1.13 | 1.13 |
| (E)/(D) | | 0.24 | 0.19 | 0.24 | 0.24 | 0.24 |
| (B)/{(D)+(E)} | | 0.037 | 0.039 | 0.037 | 0.037 | 0.037 |
| (A)/{(B)+(C)} | | 0.47 | 0.94 | 0.48 | 0.47 | 0.47 |
| Viscosity (mPa·s) | | 52.6 | 49.7 | 61.9 | 51.2 | 56.0 |
| $SP_{B+C}$ | | 20.19 | 19.82 | 20.23 | 21.40 | 19.82 |
| $\Delta(SP_A - SP_{B+C})$ *20 | | 1.58 | 1.95 1.32 6.15 | 1.54 | 0.37 | 1.95 |
| Feeling of use | Coating property to skin | 4.7 | 4.7 | 4.9 | 4.6 | 4.8 |
| | Quick-drying property on application to skin | 4.6 | 4.8 | 4.5 | 4.6 | 4.8 |
| | Appearance of coating film | 4.7 | 4.9 | 4.8 | 4.7 | 4.6 |
| | Sticky feeling of coating film | 3.6 | 4.5 | 4.6 | 4.8 | 5.0 |
| | Tense feeling | 4.5 | 4.9 | 4.6 | 4.7 | 4.8 |
| | Durability of coating film | 4.6 | 4.6 | 4.9 | 4.7 | 4.8 |
| Storage stability (0°C, one week) | | B | A | A | A | A |
| Storage stability (40°C, one week) | | C | B | A | A | A |
| Film-forming property | | 3 | 5 | 5 | 5 | 5 |
| Skin permeation amount of medicinal ingredient ($\mu g/cm^2$) | | - | - | - | - | - |
| Crystallinity evaluation of medicinal ingredient | | 5 | 4 | 5 | 5 | 5 |
| Spinnability of coating film | | 4 | 5 | 5 | 4 | 5 |
| "-" indicates that evaluation is not performed. | | | | | | |

Table 6

| (parts by mass) | | | SP value | Comparative Example | | | | | | | | | |
|---|---|---|---|---|---|---|---|---|---|---|---|---|---|
| | | | | 1 | 2 | 3 | 4 | 5 | 6 | 7 | 8 | 9 | 10 |
| (A) | *1 | Felbinac | 21.77 | 3.00 | 3.00 | 3.00 | 3.00 | 3.00 | 3.00 | | 3.00 | 3.00 | 3.00 |
| | *2 | Glycol salicylate | 26.55 | | | | | | | | | | |
| | *3 | Loxoprofen sodium | 20.18 | | | | | | | | | | |
| | *4 | l-Menthol | 18.50 | | | | | | | 3.00 | | | |
| | *5 | Nonylic acid vanillylamide | 25.97 | | | | | | | | | | |
| (B) | *6 | (Acrylates/ diacetoneacrylamide) copolymer | 21.81 | | 3.00 | 3.00 | 3.00 | 3.00 | | | 3.00 | 0.01 | 25.00 |
| | *7 | Hypromellose phthalate | 23.99 | | | | | | | | | | |
| | *8 | Acrylamide-methoxypolyethylene glycol methacrylate copolymer | 22.33 | | | | | | | | | | |
| | *9 | Ethylcellulose | 22.96 | | | | | | | | | | |
| | *10 | Polyvinyl butyral | 20.90 | | | | | | | | | | |
| (B') | *11 | Methyl vinyl ether-maleic anhydride | 28.85 | | | | | | 3.00 | 3.00 | | | |
| (C) | *12 | Isopropyl myristate | 16.29 | 1.00 | | 1.00 | 1.00 | 1.00 | 1.00 | 0.10 | | 1.00 | 1.00 |
| | *13 | Diethyl sebacate | 17.44 | | | | | | | | | | |
| | *14 | Oleyl alcohol | 18.19 | | | | | | | | | | |
| | *15 | Squalane | 15.20 | | | | | 80.60 | | | | | |
| | *16 | Diisopropanolamine | 18.46 | 2.00 | | 2.00 | 2.00 | 2.00 | 2.00 | | | 2.00 | 2.00 |
| | *17 | Triethanolamine | 28.78 | | | | | | | 2.00 | | | |
| | *18 | 2-Amino-2-methylpropanol | 22.54 | | | 0.40 | 0.40 | 0.40 | | | | 0.001 | 3.33 |

EP 4 285 931 A1

37

(continued)

| (parts by mass) | | | SP value | Comparative Example | | | | | | | | | |
|---|---|---|---|---|---|---|---|---|---|---|---|---|---|
| | | | | 1 | 2 | 3 | 4 | 5 | 6 | 7 | 8 | 9 | 10 |
| (D) | | Ethanol | | 65.00 | 65.00 | | 80.60 | | 65.00 | 65.00 | 65.00 | 65.00 | 54.00 |
| | | Isopropyl alcohol | | | | | | | | | | | |
| (E) | | Purified water | | balance | balance | balance | | | balance | balance | balance | balance | balance |
| Other | *19 | Xylitol | 36.28 | | | | | | | | 3.00 | | |
| Total amount | | | | 90.00 | 90.00 | 90.00 | 90.00 | 90.00 | 90.00 | 90.00 | 90.00 | 90.00 | 90.00 |
| | | | | Comparative Example | | | | | | | | | |
| | | | | 1 | 2 | 3 | 4 | 5 | 6 | 7 | 8 | 9 | 10 |
| Component (A) content (% by mass) | | | | 3.33 | 3.33 | 3.33 | 3.33 | 3.33 | 3.33 | 3.33 | 3.33 | 3.33 | 3.33 |
| Component (B) content (% by mass) | | | | 0.00 | 3.33 | 3.33 | 3.33 | 3.33 | 0.00 | 0.00 | 3.33 | 0.01 | 27.78 |
| Component (C) content (% by mass) | | | | 3.33 | 0.00 | 3.78 | 3.78 | 93.33 | 3.33 | 2.33 | 0.00 | 3.33 | 7.04 |
| Component (D) content (% by mass) | | | | 72.22 | 72.22 | 0.00 | 89.56 | 0.00 | 72.22 | 72.22 | 72.22 | 72.22 | 60.00 |
| Component (E) content (% by mass) | | | | 21.11 | 21.11 | 89.56 | 0.00 | 0.00 | 21.11 | 22.11 | 17.78 | 21.10 | 1.85 |
| Components (A) to (E) content (% by mass) | | | | 100.00 | 100.00 | 100.00 | 100.00 | 100.00 | 100.00 | 100.00 | 96.67 | 100.00 | 100.00 |
| (A)/(B) | | | | - | 1.00 | 1.00 | 1.00 | 1.00 | - | - | 1.00 | 300.0 | 0.12 |
| (A)/(C) | | | | 1.00 | - | 0.88 | 0.88 | 0.04 | 1.00 | 1.43 | - | 1.00 | 0.47 |
| (C)/(B) | | | | - | 0.00 | 1.13 | 1.13 | 28.00 | - | - | 0.00 | 300.13 | 0.25 |
| (E)/(D) | | | | 0.29 | 0.29 | - | 0.00 | - | 0.29 | 0.31 | 0.25 | 0.29 | 0.03 |
| (B)/{(D)+(E)} | | | | 0.000 | 0.036 | 0.037 | 0.037 | - | 0.000 | 0.000 | 0.037 | 0.0001 | 0.449 |
| (A)/{(B)+(C)} | | | | 1.00 | 1.00 | 0.47 | 0.47 | 0.03 | 1.00 | 1.43 | 1.00 | 1.00 | 0.10 |
| Viscosity (mPa·s) | | | | 0.5 | 67.8 | 102.1 | 31.5 | 114.9 | 104.1 | 40.3 | 23.9 | 0.8 | 11500 |
| $SP_{B+C}$ | | | | 17.63 | 21.81 | 19.82 | 19.82 | 15.49 | 23.00 | 28.50 | 27.55 | 17.64 | 21.46 |
| $\Delta(SP_A-SP_{B+C})$ *20 | | | | 4.14 | 0.04 | 1.95 | 1.95 | 6.28 | 1.23 | 10.00 | 5.78 | 4.13 | 0.31 |

(continued)

| | | Comparative Example | | | | | | | | | |
|---|---|---|---|---|---|---|---|---|---|---|---|
| | | 1 | 2 | 3 | 4 | 5 | 6 | 7 | 8 | 9 | 10 |
| Feeling of use | Coating property to skin | 4.2 | 4.5 | 3.5 | 2.6 | 2.4 | 4.5 | 4.4 | 4.5 | 3.7 | 2.8 |
| | Quick-drying property on application to skin | 5.0 | 4.5 | 1.6 | 4.6 | 1.7 | 4.5 | 4.4 | 4.6 | 4.8 | 2.6 |
| | Appearance of coating film | 4.8 | 4.6 | 4.4 | 4.5 | 4.7 | 4.6 | 4.5 | 4.5 | 5.0 | 3.4 |
| | Sticky feeling of coating film | 3.5 | 4.7 | 4.5 | 4.5 | 3.6 | 1.9 | 3.1 | 4.2 | 4.1 | 4.7 |
| | Tense feeling | 4.5 | 2.2 | 4.8 | 4.5 | 4.7 | 4.6 | 3.5 | 2.8 | 4.5 | 4.7 |
| | Durability of coating film | 1.4 | 4.8 | 4.3 | 3.9 | 4.2 | 2.7 | 2.8 | 4.5 | 1.9 | 4.8 |
| Storage stability (0°C, one week) | | A | A | D | B | B | B | B | A | A | D |
| Storage stability (40°C, one week) | | A | A | D | C | B | A | A | B | A | C |
| Film-forming property | | 1 | 5 | 3 | 4 | 3 | 4 | 4 | 4 | 1 | 5 |
| Skin permeation amount of medicinal ingredient ($\mu$g/cm$^2$) | | 1.83 | 0.83 | 3.54 | 4.07 | 0.97 | 5.64 | 0.05 | 0.92 | 1.93 | 2.76 |
| Crystallinity evaluation of medicinal ingredient | | 1 | 5 | 5 | 5 | 2 | 5 | 1 | 2 | 2 | 5 |
| Spinnability of coating film | | 5 | 1 | 5 | 5 | 5 | 5 | 5 | 5 | 5 | 2 |
| "-" indicates that evaluation is not performed. | | | | | | | | | | | |

Table 7

| (parts by mass) | | | SP value | Example | | | | | |
|---|---|---|---|---|---|---|---|---|---|
| | | | | 1 | 25 | 29 | 38 | 35 | 34 |
| (A) | *1 | Felbinac | 21.77 | 3.00 | 3.00 | 3.00 | 3.00 | 3.00 | 3.00 |
| | *2 | Glycol salicylate | 26.55 | | | | | | |
| | *3 | Loxoprofen sodium | 20.18 | | | | | | |
| | *4 | l-Menthol | 18.50 | | | | | | |
| | *5 | Nonylic acid vanillylamide | 25.97 | | | | | | |
| (B) | *6 | (Acrylates/diacetoneacrylamide) copolymer | 21.81 | 3.00 | 15.00 | | 3.00 | 3.00 | 3.00 |
| | *7 | Hypromellose phthalate | 23.99 | | | 15.00 | | | |
| | *8 | Acrylamide-methoxypolyethylene glycol methacrylate copolymer | 22.33 | | | | | | |
| | *9 | Ethylcellulose | 22.96 | | | | | | |
| | *10 | Polyvinyl butyral | 20.90 | | | | | | |
| (B') | *11 | Methyl vinyl ether-maleic anhydride | 28.85 | | | | | | |
| (C) | *12 | Isopropyl myristate | 16.29 | 1.00 | 1.00 | 1.00 | | 1.00 | 1.00 |
| | *13 | Diethyl sebacate | 17.44 | | | | | | |
| | *14 | Oleyl alcohol | 18.19 | | | | 1.00 | | |
| | *15 | Squalane | 15.20 | | | | | | |
| | *16 | Diisopropanolamine | 18.46 | 2.00 | 2.00 | 2.00 | 2.00 | 2.00 | 2.00 |
| | *17 | Triethanolamine | 28.78 | | | | | | |
| | *18 | 2-Amino-2-methylpropanol | 22.54 | 0.40 | 2.00 | | 0.40 | 0.40 | 0.40 |
| (D) | | Ethanol | | 65.00 | 65.00 | 65.00 | 65.00 | 50.00 | 30.00 |
| | | Isopropyl alcohol | | | | | | | |
| (E) | | Purified water | | balance | balance | balance | balance | balance | balance |
| Other | *19 | Xylitol | 36.28 | | | | | | |
| Total amount | | | | 90.00 | 90.00 | 90.00 | 90.00 | 90.00 | 90.00 |

EP 4 285 931 A1

(continued)

| | | Example | | | | | |
| --- | --- | --- | --- | --- | --- | --- | --- |
| | | 1 | 25 | 29 | 38 | 35 | 34 |
| Component (A) content (% by mass) | | 3.33 | 3.33 | 3.33 | 3.33 | 3.33 | 3.33 |
| Component (B) content (% by mass) | | 3.33 | 16.67 | 16.67 | 3.33 | 3.33 | 3.33 |
| Component (C) content (% by mass) | | 3.78 | 5.56 | 3.33 | 3.78 | 3.78 | 3.78 |
| Component (D) content (% by mass) | | 72.22 | 72.22 | 72.22 | 72.22 | 55.56 | 33.33 |
| Component (E) content (% by mass) | | 17.33 | 2.22 | 4.44 | 17.33 | 34.00 | 56.22 |
| Components (A) to (E) content (% by mass) | | 100.00 | 100.00 | 100.00 | 100.00 | 100.00 | 100.00 |
| (A)/(B) | | 1.00 | 0.20 | 0.20 | 1.00 | 1.00 | 1.00 |
| (A)/(C) | | 0.88 | 0.60 | 1.00 | 0.88 | 0.88 | 0.88 |
| (C)/(B) | | 1.13 | 0.33 | 0.20 | 1.13 | 1.13 | 1.13 |
| (E)/(D) | | 0.24 | 0.03 | 0.06 | 0.24 | 0.61 | 1.69 |
| (B)/{(D)+(E)} | | 0.037 | 0.224 | 0.217 | 0.037 | 0.037 | 0.037 |
| (A)/{(B)+(C)} | | 0.47 | 0.15 | 0.17 | 0.47 | 0.47 | 0.47 |
| Viscosity (mPa·s) | | 51.5 | 6500.0 | 9250 | 52.6 | 46.8 | 60.1 |
| $SP_{B+C}$ | | 19.82 | 21.22 | 22.86 | 20.19 | 19.82 | 19.82 |
| $\Delta(SP_A\text{-}SP_{B+C})$ *20 | | 1.95 | 0.55 | 1.09 | 1.58 | 1.95 | 1.95 |
| Feeling of use | Coating property to skin | 5.0 | 4.3 | 4.1 | 4.7 | 4.4 | 3.8 |
| | Quick-drying property on application to skin | 4.8 | 3.6 | 3.5 | 4.6 | 4.3 | 4.1 |
| | Appearance of coating film | 5.0 | 3.8 | 3.7 | 4.7 | 4.7 | 4.6 |
| | Sticky feeling of coating film | 5.0 | 4.9 | 4.8 | 3.6 | 4.5 | 4.9 |
| | Tense feeling | 4.9 | 5.0 | 4.6 | 4.5 | 4.8 | 4.6 |
| | Durability of coating film | 4.7 | 4.9 | 4.8 | 4.6 | 4.9 | 5.0 |
| Storage stability (0°C, one week) | | A | B | C | B | B | B |
| Storage stability (40°C, one week) | | A | A | A | C | A | A |
| Film-forming property | | 5 | 5 | 5 | 3 | 5 | 5 |

EP 4 285 931 A1

(continued)

| | Example | | | | | |
|---|---|---|---|---|---|---|
| | 1 | 25 | 29 | 38 | 35 | 34 |
| Skin permeation amount of medicinal ingredient ($\mu$g/cm$^2$) | 7.43 | - | 5.46 | - | - | - |
| Crystallinity evaluation of medicinal ingredient | 5 | 5 | 5 | 5 | 5 | 5 |
| Spinnability of coating film | 5 | 4 | 5 | 4 | 5 | 5 |
| Appearance change during storage at a low temperature of -5°C | Clear | Clear | Clear | Clear | Slightly cloudy | Cloudy |

The blending components and notes in the tables are as follows.

*1 Felbinac: manufactured by FUJIFILM Wako Pure Chemical Corporation
*2 Glycol salicylate: manufactured by Tokyo Chemical Industry Co., Ltd.
*3 Loxoprofen sodium: manufactured by FUJIFILM Wako Pure Chemical Corporation
*4 l-Menthol: manufactured by Takasago International Corporation
*5 Nonylic acid vanillylamide: manufactured by Tokyo Chemical Industry Co., Ltd.
*6 (Acrylates/diacetoneacrylamide) copolymer: "Plascize L-53" manufactured by Goo Chemical Co., Ltd.
*7 Hypromellose phthalate: "HPMCP HP-55" manufactured by Shin-Etsu Chemical Co., Ltd.
*8 Acrylamide-methoxypolyethylene glycol methacrylate copolymer: "RP-77T" manufactured by Kao Corporation
*9 Ethylcellulose: "ETHOCEL 100P" manufactured by Nisshin Kasei Co., Ltd.
*10 Polyvinyl butyral: "S-LEC BM-1" manufactured by Sekisui Chemical Co., Ltd.
*11 Methyl vinyl ether-maleic anhydride: "Gantrez AN" manufactured by Ashland (ISP Japan)
*12 Isopropyl myristate: "EXEPARL IPM" manufactured by Kao Corporation, specific gravity 0.80
*13 Diethyl sebacate: "Diethyl Sebacate SP-1" manufactured by NOF CORPORATION, specific gravity 0.94
*14 Oleyl alcohol: manufactured by Tokyo Chemical Industry Co., Ltd., specific gravity 0.85
*15 Squalane: manufactured by FUJIFILM Wako Pure Chemical Corporation, specific gravity 0.81
*16 Diisopropanolamine: specific gravity 0.99
*17 Triethanolamine: specific gravity 1.13
*18 2-Amino-2-methylpropanol: specific gravity 0.93
*19 Xylitol: manufactured by FUJIFILM Wako Pure Chemical Corporation, specific gravity 1.52
*20 [$\Delta(SP_A-SP_{B+C})$] in Example 39 was calculated for each kind of the component (A). $\Delta(SP_A-SP_{B+C})$ in the table is, in order from the top, $\Delta(SP_A-SP_{B+C})$ in a case where the component (A) is felbinac, l-menthol, and nonylic acid vanillylamide, respectively.

From Tables 1 to 6, it is found that the composition for external application of the present example provides good results in all of the sustained release of the medicinal ingredient, the film-forming property, the coating property to the skin, the quick-drying property, the effect of suppressing the stickiness and the tense feeling of the coating film to be formed, and the appearance and the durability of the coating film. In addition, the storage stability of the composition is also good.

In addition, from Table 7, it is found that in the composition for external application of the present example, in a case where the content of the component (E) is more preferably 50% by mass or less, still more preferably 30% by mass or less, even more preferably 25% by mass or less, even more preferably 22% by mass or less, even more preferably 21% by mass or less, and even more preferably 20% by mass or less, cloudiness hardly occurs even during low-temperature storage of -5°C, and good appearance can be maintained.

On the other hand, Comparative Examples 1, 6, and 7 which did not contain the component (B), Comparative Examples 2 and 8 which did not contain the component (C), Comparative Example 3 which did not contain the component (D), Comparative Example 4 which did not contain the component (E), Comparative Example 5 which did not contain the components (D) and (E), and Comparative Examples 9 and 10 in which the viscosity was out of the specified range of the present invention were inferior to the effects of the present invention in any of the items.

Formulation Example 1 (Preparation of Aerosol Formulation)

According to the following formulation, the component (B) was mixed with the component (C), the component (D), and the component (E) and uniformly dissolved at 30°C, and then the component (A) was mixed and dissolved to prepare a composition for external application to be an aerosol stock solution. This was filled in an aerosol container (manufactured by Toyo Seikan Co., Ltd.), and then nitrogen was sealed so that the mass ratio of aerosol stock solution:nitrogen was 1:0.1, thereby preparing an aerosol formulation.

| (Components) | (% by mass) |
|---|---|
| (A) Peppermint oil | 3.0 |
| (B) Polyvinyl alcohol | 3.0 |
| (C) Diglycerol | 3.4 |
| (D) Ethanol | 65.0 |
| (E) Purified water | balance |
| Total | 100.0 |

Industrial Applicability

According to the present invention, it is possible to provide a composition for external application which can sustainedly release a medicinal ingredient to the skin in a sustained manner, has good film-forming property, high coating property to the skin, and high quick-drying property, and is excellent in the effect of suppressing stickiness and tense feeling of a coating film to be formed, and the appearance and durability of the coating film, a formulation using the same, a method of using a composition for external application, and a method of producing a coating film.

**Claims**

1.  A composition for external application, comprising (A) a medicinal ingredient, (B) a water-insoluble polymer, (C) a non-volatile base, (D) a volatile solvent, and (E) water, wherein the composition has a viscosity at 25°C of 1.0 mPa -s or more and 10,000 mPa ·s or less.

2.  The composition for external application according to claim 1, wherein an SP value of the component (B) is 17.00 or more and 27.00 or less.

3.  The composition for external application according to claim 1 or 2, wherein an absolute value [$\Delta(SP_A-SP_{B+C})$] of a difference between an SP value ($SP_A$) of the component (A) and an SP value ($SP_{B+C}$) of a mixture of the component (B) and the component (C) is 0.10 or more and 10.00 or less.

4.  The composition for external application according to any one of claims 1 to 3, wherein the component (A) comprises one or more selected from the group consisting of an anti-inflammatory analgesic component, a local irritation component, a blood circulation promoting component, an antihistamine component, a crude drug component, an antipruritic component, a local anesthetic component, a keratin softening component, a bactericidal component, and an antibacterial component.

5.  The composition for external application according to any one of claims 1 to 4, wherein the component (A) comprises one or more selected from the group consisting of glycol salicylate, methyl salicylate, diclofenac sodium, loxoprofen sodium, ketoprofen, felbinac, indomethacin, piroxicam, flurbiprofen, glycyrrhizic acid, glycyrrhetinic acid, l-menthol, dl-camphor, nonylic acid vanillylamide, peppermint oil, benzyl nicotinate, sodium polyethylene sulfonate, tocopherol acetate, diphenhydramine, chlorpheniramine maleate, capsicum, eucalyptus oil, and phellodendron bark.

6.  The composition for external application according to any one of claims 1 to 5, wherein the component (B) is one or more water-insoluble polymers selected from the group consisting of an acrylic polymer, a cellulosic polymer, and a vinyl polymer.

7.  The composition for external application according to any one of claims 1 to 6, wherein the component (B) is one or more selected from the group consisting of (acrylates/diacetoneacrylamide) copolymer, acrylamide-methoxypolyethylene glycol methacrylate copolymer, acrylic acid-octyl acrylate copolymer, acrylate-vinyl acetate copolymer, 2-ethylhexyl acrylate-methyl acrylate-acrylic acid-glycidyl methacrylate copolymer, 2-ethylhexyl acrylate-vinyl acetate-hydroxyethyl acrylate-glycidyl methacrylate copolymer, 2-ethylhexyl acrylate-diacetoneacrylamide-acetoacetoxyethyl methacrylate-methyl methacrylate copolymer, 2-ethylhexyl acrylate-vinylpyrrolidone copolymer, 2-ethylhexyl acrylate-2-ethylhexyl methacrylate-dodecyl methacrylate copolymer, ethyl acrylate-methyl methacrylate copolymer, ethyl acrylate-methyl methacrylate-trimethylammoniumethyl methacrylate chloride copolymer, methyl methacrylate-butyl methacrylate-dimethylaminoethyl methacrylate copolymer, methylcellulose, ethylcellulose, hypromellose, carboxymethylcellulose, hydroxyethylcellulose, hydroxypropylcellulose, hypromellose phthalate, hydrophobized (C16-18) hydroxypropylmethylcellulose, polyvinyl alcohol, and polyvinyl butyral.

8.  The composition for external application according to any one of claims 1 to 7, wherein the component (C) is one or more selected from the group consisting of an ester oil, a nonpolar oil, a polyol, and an alkanolamine.

9.  The composition for external application according to any one of claims 1 to 8, wherein the component (C) is one or more selected from the group consisting of isopropyl myristate, dibutyl adipate, diethyl sebacate, diisopropyl sebacate, propylene glycol, 1,3-butylene glycol, 1,2-pentanediol, dipropylene glycol, polyethylene glycol, polypropylene glycol, polyoxyethylene/polyoxypropylene glycol, glycerol, diglycerol, triethanolamine, monoethanolamine, diisopropanolamine, and 2 -amino-2 -methylprop anol.

10. The composition for external application according to any one of claims 1 to 9, wherein the component (D) is a lower alcohol.

11. The composition for external application according to any one of claims 1 to 10, wherein a content of the component (A) in the composition for external application is 0.001% by mass or more and 30% by mass or less.

12. The composition for external application according to any one of claims 1 to 11, wherein a content of the component (B) in the composition for external application is 0.01% by mass or more and 30% by mass or less.

13. The composition for external application according to any one of claims 1 to 12, wherein a content of the component (C) in the composition for external application is 0.001% by mass or more and 30% by mass or less.

14. The composition for external application according to any one of claims 1 to 13, wherein a content of the component (D) in the composition for external application is 10% by mass or more and 95% by mass or less.

15. The composition for external application according to any one of claims 1 to 14, wherein a content of the component (E) in the composition for external application is 0.1% by mass or more and 70% by mass or less.

16. A lotion formulation, a gel formulation, an ointment formulation, a cream formulation, or a foam formulation comprising the composition for external application according to any one of claims 1 to 15.

17. An aerosol formulation or a pump spray-type formulation using the composition for external application according to any one of claims 1 to 15.

18. A method for using a composition for external application, comprising a step of applying to the skin a composition for external application comprising (A) a medicinal ingredient, (B) a water-insoluble polymer, (C) a non-volatile base, (D) a volatile solvent, and (E) water, wherein the composition has a viscosity at 25°C of 1.0 mPa ·s or more and 10,000 mPa ·s or less, and then drying the composition.

19. The method for using a composition for external application according to claim 18, wherein the composition for external application is applied to the skin by spraying.

20. A method for producing a coating film, comprising a step of applying the composition for external application according to any one of claims 1 to 15 to the skin by direct application or spraying.

# INTERNATIONAL SEARCH REPORT

International application No.

**PCT/JP2022/002876**

## A. CLASSIFICATION OF SUBJECT MATTER

*A61K 47/32*(2006.01)i; *A61K 8/34*(2006.01)i; *A61K 8/37*(2006.01)i; *A61K 8/41*(2006.01)i; *A61K 8/73*(2006.01)i; *A61K 8/81*(2006.01)i; *A61K 9/06*(2006.01)i; *A61K 9/08*(2006.01)i; *A61K 9/10*(2006.01)i; *A61K 9/107*(2006.01)i; *A61K 9/12*(2006.01)i; *A61K 31/045*(2006.01)i; *A61K 31/165*(2006.01)i; *A61K 31/192*(2006.01)i; *A61K 31/235*(2006.01)i; *A61K 31/618*(2006.01)i; *A61K 36/534*(2006.01)i; *A61K 36/61*(2006.01)i; *A61K 36/756*(2006.01)i; *A61K 36/81*(2006.01)i; *A61K 45/00*(2006.01)i; *A61K 47/10*(2006.01)i; *A61K 47/14*(2006.01)i; *A61K 47/18*(2006.01)i; *A61K 47/38*(2006.01)i; *A61P 9/08*(2006.01)i; *A61P 17/00*(2006.01)i; *A61P 17/04*(2006.01)i; *A61P 23/02*(2006.01)i; *A61P 25/02*(2006.01)i; *A61P 29/00*(2006.01)i; *A61P 31/04*(2006.01)i; *A61P 43/00*(2006.01)i; *A61Q 19/00*(2006.01)i

FI: A61K47/32; A61K8/34; A61K8/37; A61K8/41; A61K8/73; A61K8/81; A61K9/06; A61K9/08; A61K9/10; A61K9/107; A61K9/12; A61K31/045; A61K31/165; A61K31/192; A61K31/235; A61K31/618; A61K36/534; A61K36/61; A61K36/756; A61K36/81; A61K45/00; A61K47/10; A61K47/14; A61K47/18; A61K47/38; A61P9/08; A61P17/00; A61P17/04; A61P23/02; A61P25/02 101; A61P29/00; A61P31/04; A61P43/00 111; A61P43/00 113; A61Q19/00

According to International Patent Classification (IPC) or to both national classification and IPC

## B. FIELDS SEARCHED

Minimum documentation searched (classification system followed by classification symbols)

A61K47/32; A61K8/34; A61K8/37; A61K8/41; A61K8/73; A61K8/81; A61K9/06; A61K9/08; A61K9/10; A61K9/107; A61K9/12; A61K31/045; A61K31/165; A61K31/192; A61K31/235; A61K31/618; A61K36/534; A61K36/61; A61K36/756; A61K36/81; A61K45/00; A61K47/10; A61K47/14; A61K47/18; A61K47/38; A61P9/08; A61P17/00; A61P17/04; A61P23/02; A61P25/02; A61P29/00; A61P31/04; A61P43/00; A61Q19/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

## C. DOCUMENTS CONSIDERED TO BE RELEVANT

| Category* | Citation of document, with indication, where appropriate, of the relevant passages | Relevant to claim No. |
|---|---|---|
| X | JP 2020-97574 A (ROHTO PHARMACEUT. CO., LTD.) 25 June 2020 (2020-06-25) entire text, in particular, table 7, paragraph [0091] | 1-20 |
| X | JP 2020-19746 A (OSAKA SEIYAKU KK) 06 February 2020 (2020-02-06) entire text, in particular, paragraph [0028], table 1 | 1-4, 6-20 |

☑ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **16 March 2022** | **29 March 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

EP 4 285 931 A1

**INTERNATIONAL SEARCH REPORT**

| International application No. |
| --- |
| **PCT/JP2022/002876** |

**C.**     **DOCUMENTS CONSIDERED TO BE RELEVANT**

| Category* | Citation of document, with indication, where appropriate, of the relevant passages | Relevant to claim No. |
| --- | --- | --- |
| X | JP 2020-94026 A (ROHTO PHARMACEUT. CO., LTD.) 18 June 2020 (2020-06-18)<br>entire text, in particular, paragraph [0085], table 8 | 1-20 |
| X | JP 2010-248170 A (KOSE CORP.) 04 November 2010 (2010-11-04)<br>entire text, in particular, tables 5, 6 | 1-4, 6-20 |
| X | JP 2010-163419 A (KAO CORP.) 29 July 2010 (2010-07-29)<br>entire text, in particular, table 1 | 1-4, 6-20 |
| X | JP 2010-116392 A (SHISEIDO CO., LTD.) 27 May 2010 (2010-05-27)<br>entire text, in particular, example 5 | 1-4, 6-20 |
| X | JP 2010-150251 A (FUJIFILM CMIC HEALTHCARE CO., LTD.) 08 July 2010 (2010-07-08)<br>entire text, in particular, paragraph [0040], tables 1, 2, paragraph [0110] | 1-20 |
| X | JP 2010-47554 A (NIITAKA KK) 04 March 2010 (2010-03-04)<br>entire text, in particular, example 6 | 1-4, 6-20 |
| X | JP 2007-22994 A (TAISHO PHARMACEUTICAL CO., LTD.) 01 February 2007 (2007-02-01)<br>entire text, in particular, examples 1-8 | 1-6, 8-20 |
| X | JP 2006-213680 A (KOWA CO., LTD.) 17 August 2006 (2006-08-17)<br>entire text, in particular, example 1 | 1-20 |
| X | JP 2002-212105 A (LION CORP.) 31 July 2002 (2002-07-31)<br>entire text, in particular, examples 41-44 | 1-20 |
| X | JP 6-199700 A (TOKO YAKUHIN KOGYO KK) 19 July 1994 (1994-07-19)<br>entire text, in particular, examples 6-8, 10, 14 | 1-4, 6-20 |
| P, X | JP 2021-50191 A (ROHTO PHARMACEUT. CO., LTD.) 01 April 2021 (2021-04-01)<br>entire text, in particular, paragraph [0094], tables 10, 11 | 1-20 |

Form PCT/ISA/210 (second sheet) (January 2015)

INTERNATIONAL SEARCH REPORT
Information on patent family members

International application No.

**PCT/JP2022/002876**

| Patent document cited in search report | | | Publication date (day/month/year) | Patent family member(s) | | | Publication date (day/month/year) |
|---|---|---|---|---|---|---|---|
| JP | 2020-97574 | A | 25 June 2020 | (Family: none) | | | |
| JP | 2020-19746 | A | 06 February 2020 | (Family: none) | | | |
| JP | 2020-94026 | A | 18 June 2020 | (Family: none) | | | |
| JP | 2010-248170 | A | 04 November 2010 | (Family: none) | | | |
| JP | 2010-163419 | A | 29 July 2010 | (Family: none) | | | |
| JP | 2010-116392 | A | 27 May 2010 | US 2012/0022160 A1 in particular, example 5 WO 2010/044261 A1 KR 10-2011-0046585 A CN 102186454 A | | | |
| JP | 2010-150251 | A | 08 July 2010 | (Family: none) | | | |
| JP | 2010-47554 | A | 04 March 2010 | (Family: none) | | | |
| JP | 2007-22994 | A | 01 February 2007 | (Family: none) | | | |
| JP | 2006-213680 | A | 17 August 2006 | (Family: none) | | | |
| JP | 2002-212105 | A | 31 July 2002 | (Family: none) | | | |
| JP | 6-199700 | A | 19 July 1994 | US 5750579 A entire text, in particular, examples 6-8, 10, 14 EP 604848 A2 CN 1090126 A | | | |
| JP | 2021-50191 | A | 01 April 2021 | (Family: none) | | | |

Form PCT/ISA/210 (patent family annex) (January 2015)

48

## REFERENCES CITED IN THE DESCRIPTION

**Patent documents cited in the description**

- JP 2009519956 A
- JP 2014515365 A

- WO 2018194140 A